# EUROPEAN PATENT APPLICATION

(11) **EP 4 015 506 A2**
(43) Date of publication of application: **22.06.2022**
(21) Application number: 20867743.5
(22) Date of filing: 24.09.2020
(51) Int. Cl.: C07D 231/12, A61K 31/415, A61K 41/00, A61P 35/00

(54) **COMPOSITION FOR PREVENTING OR TREATING CANCER, CONTAINING NOVEL TRIFLUOROMETHYL PHENYL PYRAZOLE DERIVATIVE AS ACTIVE INGREDIENT**

(30) Priority: 25.09.2019 KR 20190118142; 23.09.2020 KR 20200122837
(71) Applicant: Korea Institute of Radiological & Medical Sciences, Seoul 01812 (KR); Korea Research Institute of Chemical Technology, Daejeon 34114 (KR)
(72) Inventor: KIM, Jae-Sung, Namyangju-si Gyeonggi-do 12095 (KR); KIM, Ah Young, Seoul 04106 (KR); KANG, Minsung, Seoul 01911 (KR); JEONG, Kwan-Young, Daejeon 34069 (KR); JO, Seokjun, Iksan-si Jeollabuk-do 54559 (KR)
(74) Representative: Roos, Peter
(86) International application number: PCT/KR2020/013001
(87) International publication number: WO 2021/060888

(57) **Abstract**

The present invention relates to a composition for preventing or treating cancer, containing a novel trifluoromethyl phenyl pyrazole derivative as an active ingredient, and, more specifically, to a composition for preventing, alleviating or treating cancer and a radiosensitizer composition for treating cancer with radiotherapy, which contain, as an active ingredient, a novel sulfonamide derivative including N,N-dimethyl-N'-(3-(1-(4-(trifluoromethyl)phenyl)-1H-pyrazol-4-yl)phenyl)azanesulfonamide. Novel sulfonamide derivatives according to the present invention have an excellent apoptotic effect on cancer cells, and thus are usable as an effective anti-cancer agent, and reduce the radioresistance of cancer cells, and thus can be used as a radiosensitizer composition during cancer treatment.

## Description

### TECHNICAL FIELD

The present invention relates to a composition for preventing or treating cancer, containing a novel trifluoromethyl phenyl pyrazole derivative as an active ingredient, and, more specifically, to a composition for preventing, alleviating, or treating cancer and a radiosensitizer composition for treating cancer with radiotherapy, which contain, as an active ingredient, a novel trifluoromethyl phenyl pyrazole derivative including N,N-dimethyl-N'-(3-(1-(4-(trifluoromethyl)phenyl)-1H-pyrazol-4-yl)phenyl)azanesulfonamide.

### BACKGROUND ART

An effective and traditional treatment method for diseases caused by the failure of normal gene control, typically referred to as cancer, is surgical excision and removal of a tumor. However, in the case that primary cancer metastasizes to other organs, surgery is not possible, so anticancer drug therapy is widely used. An anti-cancer agent used for the drug therapy is mainly used by synthesizing a single molecular substance by an organic or inorganic method. This type of traditional drug therapy is accompanied by many side effects, one of which is that the substance used as a drug is an artificially synthesized ex vivo substance and that the action point of an anticancer substance targets already overexpressed proteins.

'Cancer' refers to a group of diseases characterized by excessive cell proliferation and invasion into surrounding tissues in the case that the normal balance of apoptosis is disrupted. Among them, breast cancer in particular accounts for 25.2% incidence rate of female cancers worldwide, and the incidence rate of breast cancer between 2008 and 2012 continued to rise and increased by about 20%. In Korea, the incidence rate is 52.1%, which is one of the highest rates of breast cancer among OECD countries. It is widely known that treatment methods for breast cancer differ depending on the expression of receptors that respond to hormones. In order to treat such breast cancer, surgery, chemotherapy, radiation therapy, and hormone therapy are used in parallel depending on the progression.

Until now, many natural and protein or peptide anti-cancer agent and chemical synthetic anti-cancer agent have been developed and used, but most of them not only show serious side effects that affect normal cells in vivo, but also usually do not have the same therapeutic effect depending on the type of cancer or depending on the patient even for the same type of cancer.

In particular, as a method for treating cancer, radiation therapy is an essential treatment method for various cancers, but acquisition of radiation resistance of cancer cells and damage to normal tissues during high-dose radiation therapy are continuously pointed out as problems that reduce the efficiency of radiation therapy. Accordingly, there is an increasing need for substances capable of increasing the effect of radiation therapy in the method for treating cancer cells.

Therefore, as a new concept anticancer drug that solves the above problems worldwide, there is a need for a lot of research to develop an anticancer drug that can selectively remove only cancer cells without affecting normal cells in vivo, and further increase the sensitivity of cancer cells to radiation.

### DISCLOSURE

### TECHNICAL PROBLEM

It is an object of the present invention to provide a novel trifluoromethyl phenyl pyrazole derivative compound, an isomer thereof, or a pharmaceutically acceptable salt thereof.

In addition, another object of the present invention is to provide a pharmaceutical composition for preventing or treating cancer and a health functional food composition for preventing or alleviating cancer, containing a novel trifluoromethyl phenyl pyrazole derivative compound, an isomer thereof or a pharmaceutically acceptable salt thereof as an active ingredient.

In addition, another object of the present invention is to provide a radiosensitizer pharmaceutical composition for treating cancer with radiotherapy, containing a novel trifluoromethyl phenyl pyrazole derivative compound, an isomer thereof, or a pharmaceutically acceptable salt thereof as an active ingredient.

### TECHNICAL SOLUTION

In order to achieve the above objects, the present invention provides a compound represented by the following Formula 1, an isomer thereof, or a pharmaceutically acceptable salt thereof.

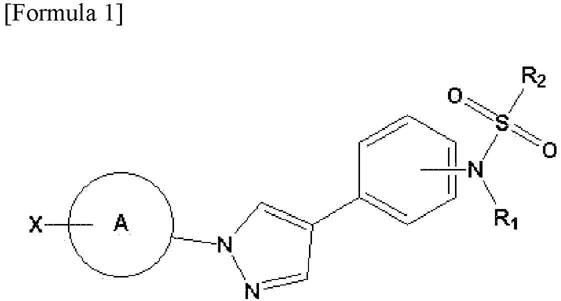

In Formula 1,
R₁ is selected from hydrogen, (C1~C6)alkyl, (C1~C6)alkene, (C1~C6)alkyne, substituted or unsubstituted aryl(C1~C6)alkyl, or substituted or unsubstituted aryl wherein said aryl may be substituted with 1 to 3 substituents which are independently selected from the group consisting of halo selected from the group consisting of fluoro, chloro and bromo, nitro, cyano, hydroxy, and oxo,
R₂ is selected from hydrogen, (C1~C6)alkyl, amino, (C1~C6)alkylamino, or di(C1~C6)alkylamino,
A is any one of aryl, pyridine, thiophene, or biphenyl, and
X is hydrogen, CF₃, CHF₂, CH₂F, (C1~C6)alkyl, (C1~C6)alkoxy, or halo selected from the group consisting of fluoro, chloro, and bromo.

Further, the present invention provides a compound represented by the following Formula 2, an isomer thereof, or a pharmaceutically acceptable salt thereof.

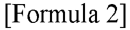

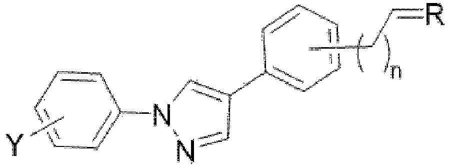

In Formula 2,
Y is hydrogen, CF₃, CHF₂, CH₂F, (C1~C6)alkyl, (C1~C6)alkoxy, or halo selected from the group consisting of fluoro, chloro, and bromo,
n is an integer between 0 to 5,
R is 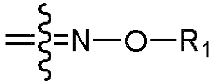 or 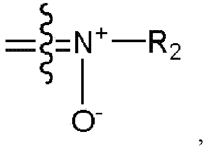 and R₁ and R₂ are each independently selected from hydrogen or substituted or unsubstituted (C1~C6)alkyl, wherein said alkyl may be substituted with 1 to 3 substituents which are independently selected from halo selected from the group consisting of fluoro, chloro, and bromo, or hydroxy.

Further, the present invention provides a pharmaceutical composition for preventing or treating cancer, containing the compound, an isomer thereof, or a pharmaceutically acceptable salt thereof as an active ingredient.

Further, the present invention provides a health functional food composition for preventing or alleviating cancer, containing the compound, an isomer thereof, or a pharmaceutically acceptable salt thereof as an active ingredient.

In addition, the present invention provides a radiosensitizer pharmaceutical composition for treating cancer with radiotherapy, containing the compound, an isomer thereof, or a pharmaceutically acceptable salt thereof as an active ingredient.

### ADVANTAGEOUS EFFECTS

The present invention relates to a composition for preventing or treating cancer, containing a novel trifluoromethyl phenyl pyrazole derivative as an active ingredient, and, more specifically, to a composition for preventing, alleviating or treating cancer and a radiosensitizer composition for treating cancer with radiotherapy, which contain, as an active ingredient, a novel sulfonamide derivative including N,N-dimethyl-N'-(3-(1-(4-(trifluoromethyl)phenyl)-1H-pyrazol-4-yl)phenyl)azanesulfonamide. Novel sulfonamide derivatives according to the present invention can be used as an effective anticancer agent because of their excellent apoptosis effect on cancer cells, and can be used as a radiosensitizer composition for cancer treatment by lowering radioresistance of cancer cells.

### BRIEF DESCRIPTIONS OF DRAWINGS

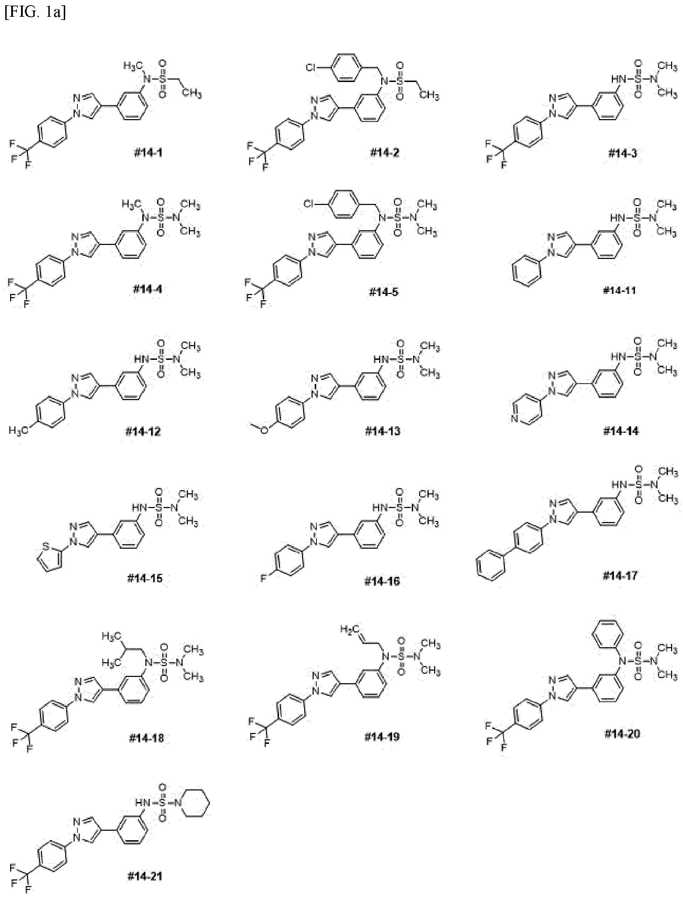

FIGS. 1a and 1b show structural formulas of compounds used in the present invention.
FIG. 2a shows results of determining anti-cancer effect of various compounds in an MCF7 breast cancer cell line. A was treated with 10 µM and B was treated with 20 µM.
FIG. 2b shows anti-cancer effect of compounds in various cancer cell lines including colorectal cancer (DLD1, HCT116), lung cancer (H1299, A545), brain cancer (U373, LN18), and the like.

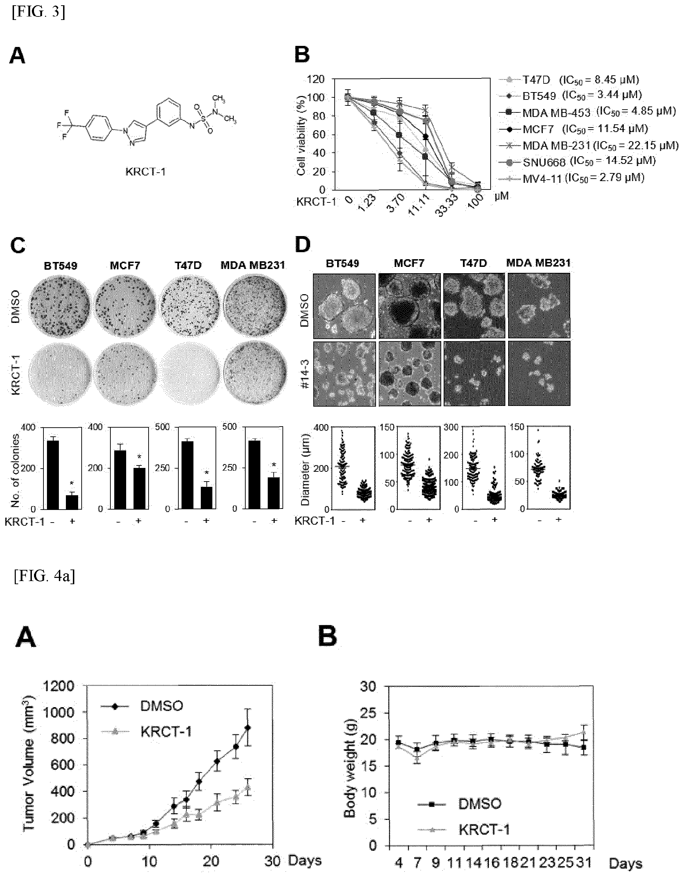

FIG. 3A shows a chemical formula of N,N-dimethyl-N'-(3-(1-(4-(trifluoromethyl)phenyl)-1H-pyrazol-4-yl)phenyl)azanesulfonamide (#14-3) designated as KRCT-1 in the present invention. FIG. 3B shows inhibition rates of cancer cell growth in the case that various breast cancer cell lines of T47D, BT-549, MDA-MB 453, MCF7, and MDA-MB 231, a stomach cancer cell line (SNU668), and a hematological cancer cell line (MV4-11) are treated with KRCT-1. FIG. 3C shows results of cancer cell line colony formation assay in the case that various breast cancer cell lines of T47D, BT-549, MCF7, and MDA-MB 231 are treated with KRCT-1. FIG. 3D shows results of tumorsphere formation assay in the case that various breast cancer cell lines of T47D, BT-549, MCF7, and MDA-MB 231 are treated with KRCT-1.
FIG. 4a shows results of monitoring tumor volume and mouse weight according to administration of KRCT-1 to an immunodeficient mouse tumor model (Xenograft mouse model) transplanted with the BT-549 cell line.
FIG. 4b shows a tumor growth inhibitory effect by KRCT-1 in the immunodeficient mouse tumor model transplanted with the BT-549 cell line. It is the result of checking the expression of a biomarker Ki-67 protein that determines a proliferative ability in a tumor dissected from the mouse shown in FIG. 4a.
FIG. 5a shows an apoptosis-inducing effect of KRCT-1 treatment in the breast cancer cell line shown by FACS analysis with Annexin V / PI staining.
FIG. 5b shows results of determining the apoptosis effect of KRCT-1 treatment in each breast cancer cell line of BT549 (A), T47D (B), and MCF7 (C) by Western blot.
FIG. 6a shows results of checking cancer cell colony formation of breast cancer cell lines BT549, T47D, and MCF7 in order to determine radiosensitivity of KRCT-1.
FIG. 6b shows results of checking DNA damage of breast cancer cell lines BT549, T47D, and MCF7 in order to determine the radiosensitivity of KRCT-1. DNA damage markers were confirmed by Western blot and immunocytochemistry (ICC).

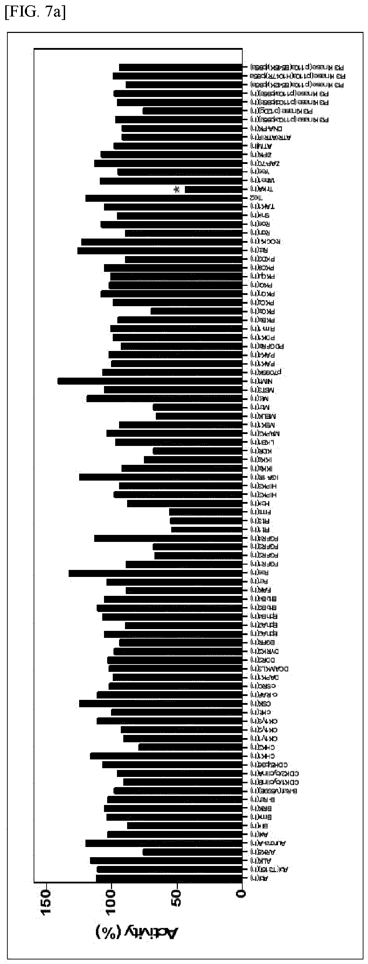

FIG. 7a shows results of 104 kinase profiling to explore a molecular biological mechanism of action of KRCT-1.

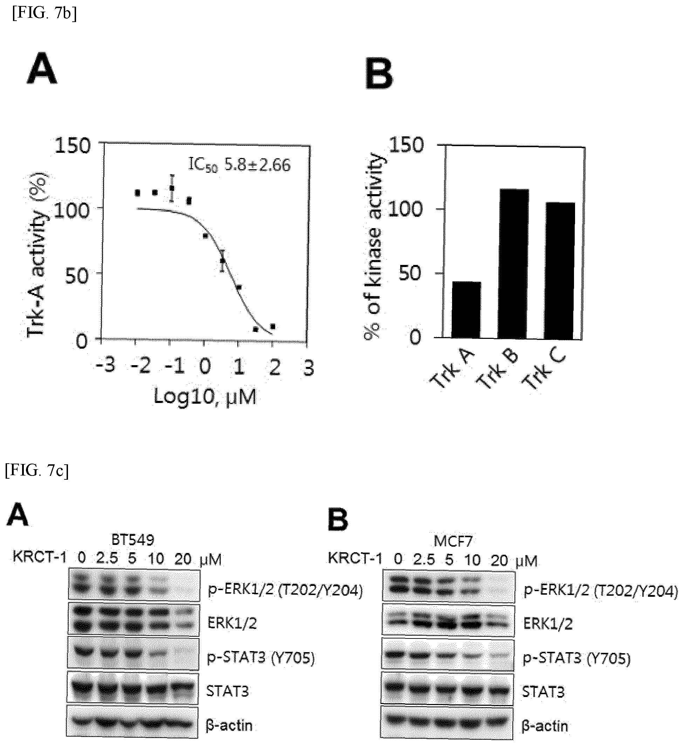

FIG. 7b shows inhibition of an in vitro Trk-A kinase activity of KRCT-1.
FIG. 7c shows results confirming that downstream signaling of Trk-A kinase in cancer cells is inhibited by KRCT-1.

### BEST MODE

The present invention provides a compound represented by the following Formula 1, an isomer thereof, or a pharmaceutically acceptable salt thereof.

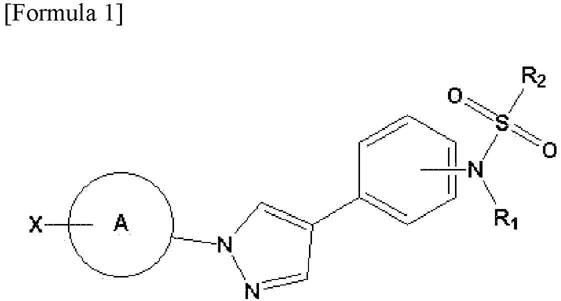

In Formula 1,
R₁ is selected from hydrogen, (C1~C6)alkyl, (C1~C6)alkene, (C1~C6)alkyne, substituted or unsubstituted aryl(C1~C6)alkyl, or substituted or unsubstituted aryl wherein said aryl may be substituted with 1 to 3 substituents which are independently selected from the group consisting of halo selected from the group consisting of fluoro, chloro, and bromo, nitro, cyano, hydroxy, and oxo,
R₂ is selected from hydrogen, (C1~C6)alkyl, amino, (C1~C6)alkylamino, or di(C1~C6)alkylamino,
A is any one of aryl, pyridine, thiophene, or biphenyl, and
X is hydrogen, CF₃, CHF₂, CH₂F, (C1~C6)alkyl, (C1~C6)alkoxy, or halo selected from the group consisting of fluoro, chloro, and bromo.
Preferably, the compound represented by Formula 1 is,
N-methyl-N-(3-(1-(4-(trifluoromethyl)phenyl)-1H-pyrazol-4-yl)phenyl)ethanesulfonamide (#14-1),
N-(4-chlorobenzyl)-N-(3-(1-(4-(trifluoromethyl)phenyl)-1H-pyrazol-4-yl)phenyl)ethanesulfonamide (#14-2),
N,N-dimethyl-N'-(3-(1-(4-(trifluoromethyl)phenyl)-1H-pyrazol-4-yl)phenyl)azanesulfonamide (#14-3),
N,N-dimethyl-N'-methyl-N'-(3-(1-(4-(trifluoromethyl)phenyl)-1H-pyrazol-4-yl)phenyl)azanesulfonamide (#14-4),
N,N-dimethyl-N'-(4-chlorobenzyl)-N'-(3-(1-(4-(trifluoromethyl)phenyl)-1H-pyrazol-4-yl) phenyl)azanesulfonamide (#14-5),
N,N-dimethyl-N '-(3-(1-(4-phenyl)-1H-pyrazol-4-yl)phenyl)azanesulfonamide (#14-11),
N,N- dimethyl-N'-(3-(1-(4-(methyl)phenyl)-1H-pyrazol-4-yl)phenyl)azanesulfonamide (#14-12),
N,N -dimethyl-N'-(3-(1-(4-(methoxy)phenyl)-1H-pyrazol-4-yl)phenyl)azanesulfonamide (#14-13),
N,N-dimethyl -N'-(3-(1-(pyridin-4-yl)-1H-pyrazol-4-yl)phenyl)azanesulfonamide (#14-14),
N,N- dimethyl-N'-(3-(1-(thiophen-4-yl)-1H-pyrazol-4-yl)phenyl)azanesulfonamide (#14-15),
N,N-dimethyl -N'-(3-(1-(4-fluorophenyl)-1H-pyrazol-4-yl)phenyl)azanesulfonamide (#14-16),
N,N-dimethyl-N'-(3-(1-(4-([1,1'-biphenyl]-1H-pyrazol-4-yl)phenyl)azanesulfonamide (#14-17),
N,N- dimethyl-N'-isobutyl-N'-(3-(1-(4-(trifluoromethyl)phenyl)-1H-pyrazol-4-yl)phenyl)azanesulfonamide (#14-18),
N,N-dimethyl-N'- allyl-N'-(3-(1-(4-(trifluoromethyl)phenyl)-1H-pyrazol-4-yl)phenyl)azanesulfonamide (#14-19),
N,N-dimethyl-N'-phenyl-N'- (3-(1-(4-(trifluoromethyl)phenyl)-1H-pyrazol-4-yl)phenyl)azanesulfonamide (#14-20), or
N-(3-(1-(4-(trifluoromethyl)phenyl)-1H-pyrazol-4-yl)phenyl)piperidine -1-sulfonamide (#14-21), but is not limited thereto.

Chemical structural formulas of compounds #14-1 to #14-5 and #14-11 to #14-21 are as follows.

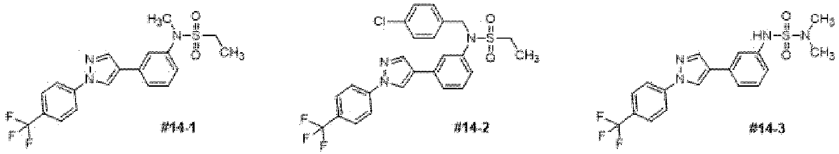

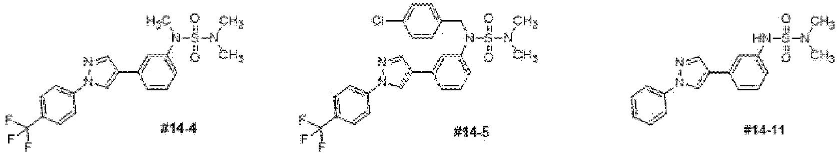

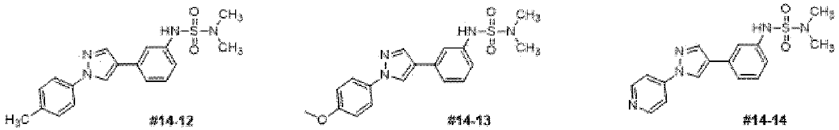

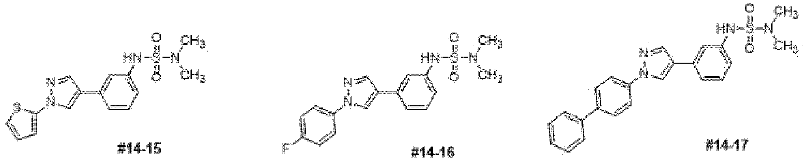

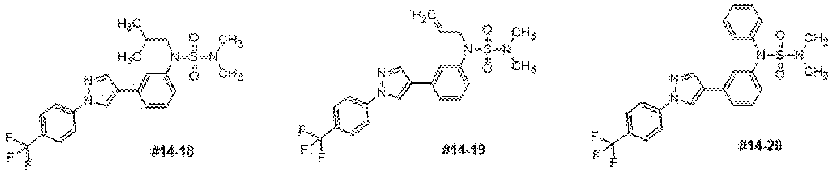

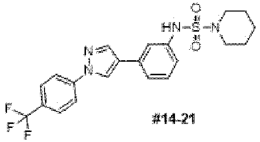

In addition, the present invention provides a compound represented by the following Formula 2, an isomer thereof, or a pharmaceutically acceptable salt thereof.

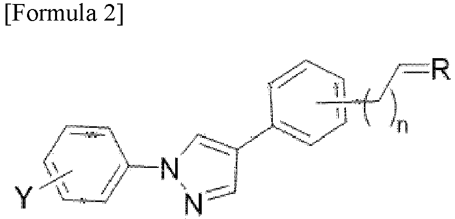

In Formula 2,
Y is hydrogen, CF₃, CHF₂, CH₂F, (C1~C6)alkyl, (C1~C6)alkoxy, or halo selected from the group consisting of fluoro, chloro, and bromo,
n is an integer between 0 to 5,
R is 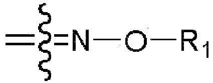 or 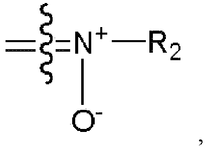 and R₁ and R₂ are each independently selected from hydrogen or substituted or unsubstituted (C1~C6)alkyl, wherein said alkyl may be substituted with 1 to 3 substituents which are independently selected from halo selected from the group consisting of fluoro, chloro, and bromo, or hydroxy.

Preferably, the compound represented by Formula 2 is,
(E)-N-ethyl-1-(3-(1-(4-(trifluoromethyl)phenyl)-1H-pyrazol-4-yl)phenyl)methanimine oxide (#14-6),
(E)-N-isopropyl-1-(3-(1-(4-(trifluoromethyl)phenyl)-1H-pyrazol-4-yl)phenyl)methanimine oxide (#14-7),
(E)-N-tert-butyl-1-(3-(1-(4-(trifluoromethyl)phenyl)-1H-pyrazol-4-yl)phenyl)methanimine oxide (#14-8),
(E)-3-(1 -(4-(trifluoromethyl)phenyl)-1H-pyrazol-4-yl)benzaldehyde O-ethyl oxime (#14-9), or
(E)- 3-(1-(4-(trifluoromethyl)phenyl)-1H-pyrazol-4-yl)benzaldehyde O-(tert-butyl) oxime (#14-10), but is not limited thereto.

Chemical structural formulas of compounds #14-6 to #14-10 are as follows.

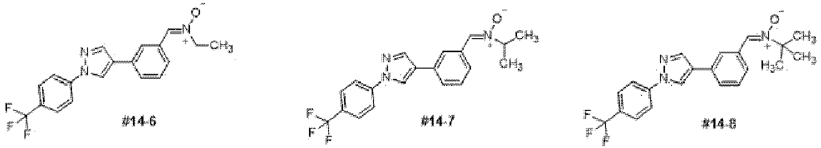

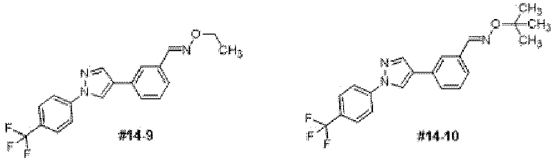

The pharmaceutically acceptable salt may be selected from the group consisting of hydrochloride, bromate, sulfate, phosphate, nitrate, citrate, acetate, lactate, tartrate, maleate, gluconate, succinate, formate, trifluoroacetate, oxalate, fumarate, methanesulfonate, benzenesulfonate, paratoluenesulfonate, camphorsulfonate, sodium salt, potassium salt, lithium salt, calcium salt, and magnesium salt, but is not limited thereto.

In addition, the present invention provides a pharmaceutical composition for preventing or treating cancer, containing the compound, an isomer thereof, or a pharmaceutically acceptable salt thereof as an active ingredient.

It was confirmed that the pharmaceutical composition of the present invention reduced the expression of survivin essential for the survival of cancer cells and induces cell death. Survivin shows a high expression rate in various cancers such as lung cancer, breast cancer, prostate cancer, and ovarian cancer, and is a protein that inhibits cell death and is involved in the survival and proliferation of cancer cells. The survivin is being studied a lot as a target for the development of diagnostic markers and targeted anti-cancer agent. Accordingly, the present inventors determined that cancer cell proliferation could be effectively inhibited in the case that cancer cells were treated with KRCT-1, which inhibited the expression of survivin.

In the case that the composition of the present invention is a pharmaceutical composition, it may be prepared using a pharmaceutically suitable and physiologically acceptable adjuvant in addition to the active ingredient, and as the adjuvant, an excipient, a disintegrant, a sweetener, a binder, a coating agent, a blowing agent, a lubricant, a glidant agent, a flavoring agent, or a solubilizer may be used. The pharmaceutical composition of the present invention may be preferably formulated into a pharmaceutical composition including one or more pharmaceutically acceptable carriers in addition to the active ingredient for administration. In the composition formulated as a liquid solution, acceptable pharmaceutical carriers are sterile and biocompatible, and may be used by mixing saline, sterile water, Ringer's solution, buffered saline, albumin injection, dextrose solution, maltodextrin solution, glycerol, ethanol and one or more of these components, and other conventional additives such as antioxidants, buffers, and bacteriostats may be added as needed. In addition, diluents, dispersants, surfactants, binders and lubricants may be additionally added to formulate an injectable formulation such as an aqueous solution, suspension, and emulsion, pills, capsules, granules, or tablets.

The pharmaceutical formulation form of the pharmaceutical composition of the present invention may be granules, powders, coated tablets, tablets, capsules, suppositories, syrups, juices, suspensions, emulsions, drops or injectable solutions, and sustained-release formulations of the active compound. The pharmaceutical composition of the present invention can be administered in a conventional manner via intravenous, intraarterial, intraperitoneal, intramuscular, intrasternal, transdermal, intranasal, inhalational, topical, rectal, oral, intraocular or intradermal routes. The effective amount of the active ingredient of the pharmaceutical composition of the present invention means an amount required for preventing or treating a disease. Therefore, it may be adjusted according to various factors, including the type of disease, the severity of the disease, the type and content of the active ingredient and other ingredients contained in the composition, the type of formulation and the age, weight, general health condition, sex and diet of the patient, administration time, administration route and composition secretion rate, duration of treatment, and concomitant drugs.

In addition, the pharmaceutical composition according to the present invention may contain a trifluoromethyl phenyl pyrazole derivative compound at a concentration of 5 to 150 µM as an active ingredient, preferably 10 to 120 µM, and it may be included more preferably at an effective concentration of 30 to 100 µM.

In the present invention, 'cancer' refers to a group of diseases characterized by excessive cell proliferation and infiltration into surrounding tissues in the case that the normal balance of apoptosis is disrupted. The cancer of the present invention may be selected from the group consisting of brain tumor, benign astrocytoma, malignant astrocytoma, pituitary adenoma, meningioma, brain lymphoma, oligodendroglioma, intracranial tumor, ependymoma, brain stem tumor, head and neck tumor, laryngeal cancer, oropharyngeal cancer, nasal/sinus cancer, nasopharyngeal cancer, salivary gland cancer, hypopharyngeal cancer, thyroid cancer, chest tumor, small cell lung cancer, non-small cell lung cancer, thymus cancer, mediastinum tumor, esophageal cancer, breast cancer, male breast cancer, abdominal tumor, stomach cancer, liver cancer, gallbladder cancer, biliary tract cancer, pancreatic cancer, small intestine cancer, colorectal cancer, anal cancer, bladder cancer, kidney cancer, male genital tumor, penile cancer, urethral cancer, prostate cancer, female genital tumor, cervical cancer, endometrial cancer, ovarian cancer, uterine sarcoma, vaginal cancer, external genital cancer, female urethra cancer, skin cancer, myeloma, leukemia, and malignant lymphoma, preferably may be breast cancer, prostate cancer, lung cancer, or stomach cancer, and most preferably, breast cancer, but is not limited thereto.

The pharmaceutical composition of the present invention can be used in combination with radiation therapy, and in particular, the pharmaceutical composition of the present invention can increase the sensitivity to radiation even for cancer cells or cancer cells having radioresistance, so in the case that it can be used in combination with radiation therapy, in a method of treating cancer cells, it is characterized in that it can increase the effect of radiation therapy.

In addition, the present invention provides a health functional food composition for preventing or alleviating cancer, containing the compound, an isomer thereof, or a pharmaceutically acceptable salt thereof as an active ingredient.

As used herein, the term "health functional food" refers to a food manufactured and processed using a specific ingredient as a raw material for the purpose of health supplementation or by extracting, concentrating, refining, or mixing a specific ingredient contained in the food raw material. It refers to food that is designed and processed to sufficiently exert biological control functions, such as biological defense, regulation of biological rhythm, and prevention and recovery of disease, by the components and refers to a food that can perform functions related to prevention of disease or restoration of health.

In the case that the trifluoromethyl phenyl pyrazole derivative compound according to the present invention is used as a health functional food, it may be added as it is or used together with other foods or food ingredients, which may be appropriately selected as needed.

In addition, there is no particular limitation on the type of health functional food in which the trifluoromethyl phenyl pyrazole derivative compound according to the present invention can be used. For example, there are ramen, other noodles, beverages, tea, drinks, alcoholic beverages, various soups, meat, sausage, bread, chocolate, candy, confectionery, pizza, gum, dairy products, including ice cream, or vitamin complex. In addition, the health functional food according to the present invention may be mixed with other suitable auxiliary ingredients and known additives that may be normally contained in health functional food according to the selection of those skilled in the art other than the trifluoromethyl phenyl pyrazole derivative compound.

In addition, the present invention provides a radiosensitizer pharmaceutical composition for treating cancer with radiotherapy, containing the compound, an isomer thereof, or a pharmaceutically acceptable salt thereof as an active ingredient.

In the case that the trifluoromethyl phenyl pyrazole derivative compound of the present invention is treated with cancer cells, the sensitivity of the cancer cells to radiation is increased, thereby enhancing the death of cancer cells.

The criteria for radiosensitivity or radioresistance can be classified according to the slope value of a linear model created using cell viability in the case that irradiated with 0 to 3 Gy of gamma radiation. This classification method has been reported by Jerry R. Williams et al. in their papers (Acta Oncologica, 46, 628-638, 2007). As used herein, the term "radiosensitivity" means a cell having a characteristic that the slope value is in the range of 0.00 to 0.30, and "radioresistance" refers to a cell having a characteristic included in the range of 0.31 to 1.00.

The cancers of the present invention may be selected from the group consisting of brain tumor, benign astrocytoma, malignant astrocytoma, pituitary adenoma, meningioma, brain lymphoma, oligodendroglioma, intracranial tumor, ependymoma, brain stem tumor, head and neck tumor, laryngeal cancer, oropharyngeal cancer, nasal/sinus cancer, nasopharyngeal cancer, salivary gland cancer, hypopharyngeal cancer, thyroid cancer, chest tumor, small cell lung cancer, non-small cell lung cancer, thymus cancer, mediastinum tumor, esophageal cancer, breast cancer, male breast cancer, abdominal tumor, stomach cancer, liver cancer, gallbladder cancer, biliary tract cancer, pancreatic cancer, small intestine cancer, colorectal cancer, anal cancer, bladder cancer, kidney cancer, male genital tumor, penile cancer, urethral cancer, prostate cancer, female genital tumor, cervical cancer, endometrial cancer, ovarian cancer, uterine sarcoma, vaginal cancer, external genital cancer, female urethra cancer, skin cancer, myeloma, leukemia, and malignant lymphoma, but may preferably be prostate cancer including breast cancer, lung cancer or stomach cancer, and most preferably, breast cancer, but is not limited thereto.

The radiosensitizer pharmaceutical composition for treating cancer with radiotherapy according to the present invention may be used in combination with radiation therapy.

The radiation therapy used in the present invention is a local treatment method that damages the DNA of malignant cells. Normal cells have a greater capacity to repair this damage than tumor cells, and the radiation therapy is known to take advantage of this difference.

The radiation therapy may include external beam radiation (x-rays, γ-rays, protons and neutrons), brachytherapy, and radioactive material implantation, and may be administered by 2-D, 3-D, confocal, intensity-modulated (IMRT) and image-guided (IGRT) approaches. The standard radiation therapy that can be used in the cancer treatment of the present invention may be a method of providing a total dose of about 60 Gys (50 ~ 70 Gys) at 2.5 Gy/day. However, those skilled in the art may select the desired radiation dose based on the particular subject, device, and tumor type.

The radiosensitizer pharmaceutical composition for treating cancer with radiotherapy according to the present invention constitutes an improvement over the existing radiation therapy by increasing the sensitivity of cancer cells to radiation. For example, the use of the radiosensitizer pharmaceutical composition for treating cancer with radiotherapy of the present invention causes cancer cells to have sensitivity of at least 30%, 40%, 50%, 60%, 70%, 80%, 90%, 100% or more to radiation. As used herein, the terms "sensitivity" and "radiosensitivity" refer to the number of viable cells based on radiation dose. Accordingly, an increase in radiosensitivity may mean a decrease in the number of cells surviving at a certain radiation dose, a decrease in the radiation dose required for a lethal dose, or a combination thereof.

Since the radiosensitizer pharmaceutical composition for treating cancer with radiotherapy of the present invention can increase the sensitivity not only for radiotherapy of cancer cells but also for chemotherapy, the radiosensitizer of the present invention can be used as a sensitizer for increasing sensitivity in chemotherapy. The use case may also fall within the scope of the present invention.

As used herein, the term "administered in combination" means administering radiation irradiation together in an anticancer process for treating various types of cancer cells. Specifically, it may be one in which radiation therapy is concurrently treated in an anticancer process for treating cancer cells such as solid cancers such as lung cancer, breast cancer, colorectal cancer, ovarian cancer, head and neck cancer, and brain cancer.

### MODES FOR CARRYING OUT INVENTION

Hereinafter, the present invention will be described in detail through examples. These examples are merely for illustrating the present invention in more detail, and it will be apparent to those of skilled in the art that the scope of the present invention is not limited by these examples according to the gist of the present invention.

### <Preparation Example 1> 3-(1-(4-(trifluoromethyl)phenyl)-1H-pyrazol-4-yl)aniline

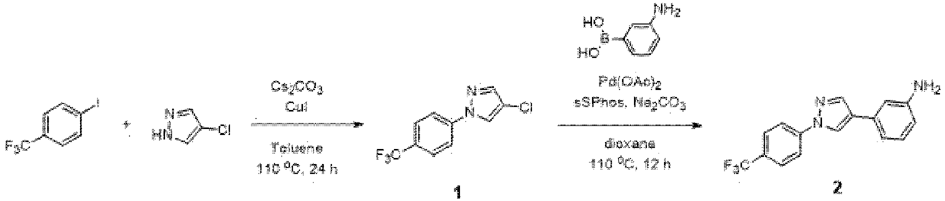

Step 1: 4-iodobenzotrifluoride (1.00 g, 3.68 mmol), 4-chloro-1H-pyrazole (415 mg, 4.05 mmol), cesium carbonate (2.40 g, 7.36 mmol), CuI (105 mg, 0.55 mmol) were added to anhydrous toluene (35 mL), which is a reaction solvent, and then stirred at 110°C for 24 hours. After confirming the reaction product by TLC, the reaction solution was diluted with EtOAc and washed with purified water. The organic layer was dehydrated over anhydrous Na₂SO₄, concentrated under reduced pressure, and separated by silica column chromatography to obtain 1.12 g of the title compound (compound 1). ¹H-NMR(300 MHz, CDCl₃): δ 8.23 (s, 1H), 8.02 (s, 1H), 7.89 (2H, d, J=8.77 Hz), 7.76 (2H, d, J=8.77 Hz); LC-MS (ESI, m/z) = 247.0 (M+H⁺).

Step 2: The compound 1 (800 mg, 3.25 mmol) obtained in step 1 was put in a reaction vessel, and 3-aminophenylboronic acid(534 mg, 3.90 mmol), Pd(OAc)₂(73 mg, 0.32 mmol), sSPhos(250 mg, 0.49 mmol), Na₂CO₃(689 mg, 6.50 mmol) were added to anhydrous dioxane (25 mL), followed by treatment with N₂(g) for 5 minutes. After the reaction was stirred at 110° C for 12 hours, the product was confirmed by TLC, and dioxane, a solvent, was removed by concentration under reduced pressure. The remaining reaction mixture was diluted with EtOAc and washed with purified water. The organic layer was dehydrated with anhydrous Na₂SO₄, concentrated under reduced pressure, and separated by silica column chromatography to obtain 921 mg of the title compound (compound 2). ¹H-NMR(300 MHz, CDCl₃): δ 8.21 (s, 1H), 8.08 (s, 1H), 7.81 (2H, d, J=8.74 Hz), 7.67 (2H, d, J=8.77 Hz), 7.45 (1H, m), 7.02 (1H, d, J=7.82 Hz), 6.89 (1H, s), 6.76 (1H, d, J=7.69 Hz), 4.66 (2H, s); LC-MS (ESI, m/z) = 304.1 (M+H⁺).

### <Preparation Example 2> 2-(1-(4-(trifluoromethyl)phenyl)-1H-pyrazol-4-yl)benzaldehyde

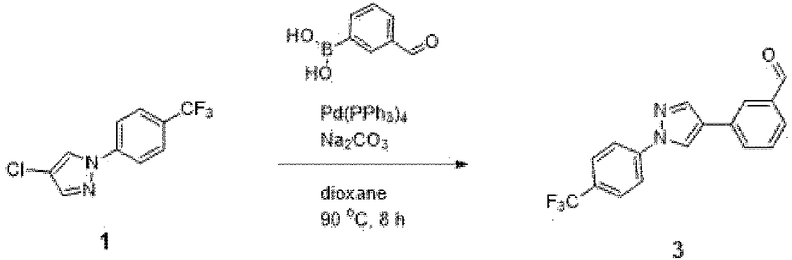

Compound 1 (800 mg, 3.25 mmol) obtained in step 1 of Preparation Example 1 was put in a reaction vessel, and (3-formylphenyl)boronic acid(585 mg, 3.90 mmol), Pd(PPh₃)₄(375 mg, 0.33 mmol), Na₂CO₃(689 mg, 6.54 mmol) was added to dioxane (32 mL), followed by treatment with N₂(g) for 5 minutes. After the reaction was stirred at 90° C for 8 hours, the product was confirmed by TLC, and dioxane, a solvent, was removed by concentration under reduced pressure. The reaction concentrate was diluted with EtOAc and washed with purified water. The organic layer was dehydrated over anhydrous Na₂SO₄, concentrated under reduced pressure, and separated by silica column chromatography to obtain 870 mg of the title compound (compound 3). ¹H-NMR(300 MHz, CDCl₃): δ 9.85 (s, 1H), 8.61 (s, 1H), 8.21 (s, 1H), 8.08 (s, 1H), 7.95 (1H, d, J=7.86 Hz), 7.81 (2H, d, J=8.74 Hz), 7.67 (2H, d, J=8.77 Hz), 7.45 (1H, m), 7.22 (1H, d, J=7.82 Hz); LC-MS (ESI, m/z) = 317.1 (M+H⁺).

### <Example 1> Compound Synthesis

### 1. #14-1 and #14-2 synthesis scheme

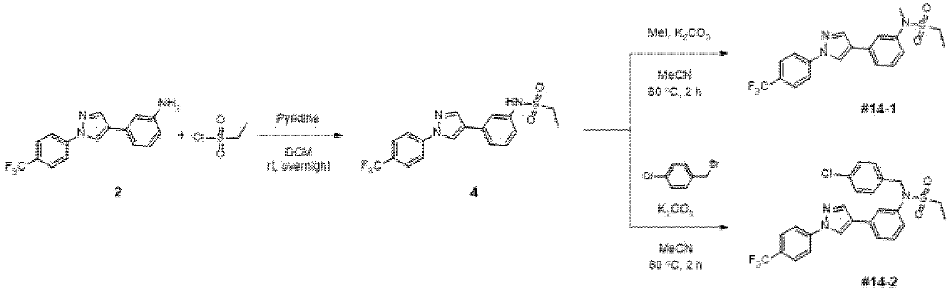

### #14-1: N-methyl-N-(3-(1-(4-(trifluoromethyl)phenyl)-1H-pyrazol-4-yl)phenyl)ethanesulfonamide

Step 1: Compound 2 synthesized in Preparation Example 1, 3-(1-(4-(trifluoromethyl)phenyl)-1H-pyrazol-4-yl)aniline (591 mg, 1.95 mmol) was added into a reaction flask, and dissolved with a 20 mL anhydrous DCM solvent. Ethanesulfonyl chloride (204 µL, 2.16 mmol) and pyridine (174 µL, 2.16 mmol) were added, followed by stirring at room temperature for 12 hours. After the reaction product was confirmed by TLC, it was diluted with DCM and washed with NH₄Cl aqueous solution. The separated organic layer was dehydrated with anhydrous Na₂SO₄, concentrated under reduced pressure, and separated by column chromatography to obtain 603 mg of compound 4. ¹H-NMR (300 MHz, CDCl₃): δ 10.51 (s, 1H), 8.20 (s, 1H), 8.04 (s, 1H), 7.83 (d, 2H, J=8.77 Hz), 7.73 (d, 2H, J= 8.77 Hz), 7.62 (s, 1H), 7.51 (d, 1H, J= 7.44 Hz), 7.42 (t, 1H, J= 7.71 Hz), 7.27 (d, 1H, J= 7.71 Hz), 3.12 (q, 2H, J= 7.44 Hz), 1.42 (t, 3H, J= 7.71 Hz); LC-MS (ESI, m/z) = 396.1 (M+H⁺).

Step 2: The compound 4 (256 mg, 0.65 mmol) obtained in step 1 above was put in a reaction flask, and dissolved with 7 mL of anhydrous MeCN solvent. Methyl iodide (38 µL, 0.65 mmol) and K₂CO₃ (90 mg, 0.65 mmol) were added, and the mixture was stirred at 80°C for 2 hours. The reaction product was confirmed by TLC, diluted with EtOAc and washed with purified water. The separated organic layer was dehydrated with anhydrous Na₂SO₄, concentrated under reduced pressure, and separated by column chromatography to obtain 220 mg of the title compound #14-1. ¹H-NMR (300 MHz, CDCl₃): δ 8.23 (s, 1H), 8.02 (s, 1H), 7.89 (d, 2H, J=8.77 Hz), 7.76 (d, 2H, J= 8.77 Hz), 7.62 (s, 1H), 7.49 (d, 1H, J= 7.44 Hz), 7.45 (t, 1H, J= 7.71 Hz), 7.30 (d, 1H, J= 7.71 Hz), 3.40 (s, 3H), 3.12 (q, 2H, J= 7.44 Hz), 1.42 (t, 3H, J= 7.71 Hz); LC-MS (ESI, m/z) = 410.2 (M+H⁺).

### #14-2: N-(4-chlorobenzyl)-N-(3-(1-(4-(trifluoromethyl)phenyl)-1H-pyrazol-4-yl)phenyl)ethanesulfonamide

Compound 4 (67 mg, 0.17 mmol) obtained in step 1 above was placed in a reaction flask and dissolved with 3 mL of anhydrous MeCN solvent. After adding 1-(Bromomethyl)-4-chlorobenzene (82 mg, 0.21 mmol) and K₂CO₃(29 mg, 0.21 mmol), the mixture was stirred at 80° C for 2 hours. The reaction product was confirmed by TLC, diluted with EtOAc, and washed with purified water. The separated organic layer was dehydrated over anhydrous Na₂SO₄,concentrated under reduced pressure, and separated by column chromatography to obtain 60 mg of the title compound #14-2. ¹H-NMR (300 MHz, CDCl₃): δ 8.12 (s, 1H), 7.94 (s, 1H), 7.87 (d, 2H, J= 8.54 Hz), 7.75 (d, 2H, J= 8.54 Hz), 7.46 (m, 1H), 7.38 (m, 2H), 7.24 (m, 3H), 7.17 (m, 1H), 4.88 (s, 2H), 3.15 (q, 2H, J= 7.35 Hz), 1.47 (t, 3H, J= 7.35 Hz); LC-MS (ESI, m/z) = 520.1 (M+H⁺).

### 2. #14-3, #14-4, #14-5, #14-11 to #14-21 synthesis scheme

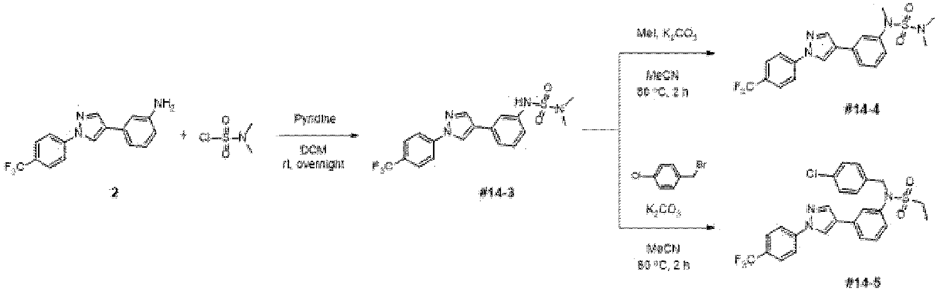

### #14-3: N,N-dimethyl-N'-(3-(1-(4-(trifluoromethyl)phenyl)-1H-pyrazol-4-yl)phenyl)azanesulfonamide

Compound 2 synthesized in Preparation Example 1, 3-(1-(4-(trifluoromethyl)phenyl)-1H-pyrazol-4-yl)aniline (591 mg, 1.95 mmol) was placed in a reaction flask, and dissolved with 20 mL of anhydrous DCM solvent. Dimethylsulfamoyl chloride (671 µL, 2.16 mmol) and pyridine (174 µL, 2.16 mmol) were added, followed by stirring at room temperature for 12 hours. After the reaction product was confirmed by TLC, it was diluted with DCM and washed with NH₄Cl solution. The separated organic layer was dehydrated over anhydrous Na₂SO₄, concentrated under reduced pressure, and separated by column chromatography to obtain 781 mg of the title compound #14-3. ¹H-NMR (300 MHz, CDCl₃): δ 8.21 (S, 1H), 8.01 (s, 1H), 7.89 (d, 2H, J= 8.51 Hz), 7.76 (d, 2H, J= 8.51 Hz), 7.40 (m, 1H), 7.36 (m, 2H), 7.09 (m, 1H), 6.54 (s, 1H), 2.89 (s, 6H); LC-MS (ESI, m/z) = 411.1 (M+H⁺).

### #14-4: N,N-dimethyl-N'-methyl-N'-(3-(1-(4-(trifluoromethyl)phenyl)-1H-pyrazol-4-yl)phenyl)azanesulfonamide

The compound of Example #14-3 (100 mg, 0.24 mmol) was placed in a reaction flask and dissolved with 3 mL of anhydrous MeCN solvent. After adding iodomethane (17 µL, 0.28 mmol) and K₂CO₃(67 mg, 0.50 mmol), the mixture was stirred at 80°C for 2 hours. After the reaction product was confirmed by TLC, it was diluted with EtOAc and washed with purified water. The separated organic layer was dehydrated over anhydrous Na₂SO₄,concentrated under reduced pressure, and separated by column chromatography to obtain 94 mg of the title compound #14-4. ¹H-NMR (300 MHz, CDCl₃): δ 8.23 (s, 1H), 8.02 (s, 1H), 7.89 (d, 2H, J= 8.62 Hz), 7.76 (d, 2H, J= 8.62 Hz), 7.62 (m, 1H), 7.48 (m, 1H), 7.44 (t, 1H, J= 7.63 Hz), 7.32 (m, 1H), 3.33 (s, 3H), 2.83 (s, 6H); LC-MS (ESI, m/z) = 425.1 (M+H⁺).

### #14-5: N,N-dimethyl-N'-(4-chlorobenzyl)-N'-(3-(1-(4-(trifluoromethyl)phenyl)-1H-pyrazol-4-yl)phenyl)azanesulfonamide

The compound of Example #14-3 (100 mg, 0.24 mmol) was placed in a reaction flask and dissolved with 3 mL of anhydrous MeCN solvent. 4-Chlorobenzyl bromide (55 mg, 0.28 mmol) and K₂CO₃ (67 mg, 0.50 mmol) were added, followed by stirring at 80°C for 2 hours. After the reaction product was confirmed by TLC, it was diluted with EtOAc and washed with purified water. The separated organic layer was dehydrated over anhydrous Na₂SO₄, concentrated under reduced pressure, and separated by column chromatography to obtain 106 mg of the title compound #14-5. ¹H-NMR (300 MHz, CDCl₃): δ 8.12 (s, 1H), 7.94 (s, 1H), 7.88 (d, 2H, J= 8.83 Hz), 7.75 (d, 2H, J= 8.83 Hz), 7.46 (m, 1H), 7.37 (t, 1H, J= 7.61 Hz), 7.26 (s, 1H), 7.24 (d, 2H, J= 10.96 Hz), 7.15 (m, 1H), 4.78 (s, 2H), 2.78 (s, 6H); LC-MS (ESI, m/z) = 535.1 (M+H⁺).

### #14-11: N,N-dimethyl-N'-(3-(1-(4-phenyl)-1H-pyrazol-4-yl)phenyl)azanesulfonamide

Step 1: Iodobenzene (1.00 g, 4.90 mmol) and 4-chloro-1H-pyrazole (552 mg, 5.39 mmol) were reacted in a similar manner to Preparation Example 1 to obtain 750 mg of the title compound 3-(1-phenyl-1H- pyrazol-4-yl)aniline. ¹H-NMR (300 MHz, CDCl₃): δ 8.19 (s, 1H), 8.10 (s, 1H), 7.64 (2H, d, J=8.71 Hz), 7.42 (2H, m), 7.02 (1H, d, J=7.69 Hz), 6.84 (1H, s), 6.78 (1H, d, J=7.74 Hz), 4.67 (2H, s); LC-MS (ESI, m/z) = 236.1 (M+H⁺).

Step 2: Using the compound obtained in step 1, 3-(1-phenyl-1H-pyrazol-4-yl)aniline (200 mg, 0.85 mmol) as a starting material for the reaction, it was reacted in a similar manner to Example #14-3 to obtaine 87 mg of the title compound. ¹H-NMR (300 MHz, CDCl₃): δ 10.51 (s, 1H), 8.19 (S, 1H), 8.01 (s, 1H), 7.87-7.74 (m, 5H), 7.40 (m, 1H), 7.36 (m, 2H), 7.12 (m, 1H), 6.52 (s, 1H), 2.89 (s, 6H); LC-MS (ESI, m/z) = 344.2 (M+H⁺).

### #14-12: N,N-dimethyl-N'-(3-(1-(4-(methyl)phenyl)-1H-pyrazol-4-yl)phenyl)azanesulfonamide

Step 1: Using 1-iodo-4-methylbenzene (1.00 g, 4.58 mmol) as a starting material, it was reacted in a manner similar to Preparation Example 1 to obtain 632 mg of the title compound 3-(1-(p-tolyl)-1H-pyrazol-4-yl)aniline. 1H-NMR (300 MHz, CDCl3): δ 8.21 (s, 1H), 8.09 (s, 1H), 7.82 (2H, d, J=8.77 Hz), 7.69 (2H, d, J=8.77 Hz), 7.00 (1H, d, J=7.85 Hz), 6.86 (1H, s), 6.76 (1H, d, J=7.66 Hz), 4.69 (s, 2H), 2.43 (s, 3H); LC-MS (ESI, m/z) = 250.08 (M+H⁺).

Step 2: Using the compound obtained in step 1, 3-(1-(p-tolyl)-1H-pyrazol-4-yl)aniline (200 mg, 0.83 mmol) as a starting material for the reaction, it was reacted in a similar manner to Example #14-3 to obtaine 58 mg of the title compound. 1H-NMR (300 MHz, CDCl₃): δ 10.50 (s, 1H), 8.20 (S, 1H), 8.00 (s, 1H), 7.89 (d, 2H, J=8.48 Hz), 7.76 (d, 2H, J=8.51 Hz), 7.37 (m, 1H), 7.38 (m, 2H), 7.08 (m, 1H), 6.54 (s, 1H), 2.44 (s, 3H), 2.88 (s, 6H); LC-MS (ESI, m/z) = 357.1 (M+H⁺).

### #14-13: N,N-dimethyl-N'-(3-(1-(4-(methoxy)phenyl)-1H-pyrazol-4-yl)phenyl)azanesulfonamide

Step 1: Using 1-iodo-4-methoybenzene (1.00 g, 4.27 mmol) as a starting material, it was reacted in a manner similar to Preparation Example 1 to obtain 734 mg of the title compound 3-(1-(4-methoxyphenyl)-1H-pyrazol-4-yl)aniline. 1H-NMR (300 MHz, CDCl3): δ 8.20 (s, 1H), 8.03 (s, 1H), 7.78 (2H, d, J=8.74 Hz), 7.64 (2H, d, J=8.74 Hz), 6.97 (1H, d, J=7.74 Hz), 6.85 (1H, s), 6.76 (1H, d, J=7.66 Hz), 4.69 (s, 2H), 3.82 (s, 3H); LC-MS (ESI, m/z) = 266.0 (M+H⁺).

Step 2: Using the compound obtained in step 1, 3-(1-(4-methoxyphenyl)-1H-pyrazol-4-yl)aniline (200 mg, 0.76 mmol) as a starting material for the reaction, it was reacted in a similar manner to Example #14-3 to obtaine 61 mg of the title compound. 1H-NMR (300 MHz, CDCl3): δ 8.15 (S, 1H), 8.03 (s, 1H), 7.85 (d, 2H, J=8.45 Hz), 7.69 (d, 2H, J=8.48 Hz), 7.34 (m, 1H), 7.30 (m, 2H), 7.09 (m, 1H), 6.52 (s, 1H), 3.81 (s, 3H), 2.88 (s, 6H); LC-MS (ESI, m/z) = 373.1 (M+H⁺).

### #14-14: N,N-dimethyl-N'-(3-(1-(pyridin-4-yl)-1H-pyrazol-4-yl)phenyl)azanesulfonamide

Step 1: Using 4-iodopyridine (1.00 g, 4.87 mmol) as a starting material, it was reacted in a manner similar to Preparation Example 1 to obtaine 541 mg of the title compound 3-(1-(pyridin-4-yl)-1H-pyrazol-4-yl)aniline. 1H-NMR (300 MHz, CDCl3): δ 8.32 (s, 1H), 8.12 (s, 1H), 7.98 (2H, d, J=8.64 Hz), 7.83 (2H, d, J=6.64 Hz), 7.01 (m, 1H), 6.97 (1H, d, J=7.72 Hz), 6.85 (1H, s), 6.72 (1H, d, J=7.72 Hz), 4.69 (s, 2H); LC-MS (ESI, m/z) = 237.0 (M+H⁺).

Step 2: Using the compound obtained in step 1, 3-(1-(pyridin-4-yl)-1H-pyrazol-4-yl)aniline (200 mg, 0.84 mmol) as a starting material for the reaction, it was reacted in a similar manner to Example #14-3 to obtaine 47 mg of the title compound. 1H-NMR (300 MHz, CDCl3): δ 10.53 (s, 1H), 8.21 (S, 1H), 8.19 (s, 1H), 7.98 (d, 2H, J=8.42 Hz), 7.79 (d, 2H, J=8.42 Hz), 7.31 (m, 1H), 7.29 (m, 2H), 7.09 (m, 1H), 6.51 (s, 1H), 2.87 (s, 6H); LC-MS (ESI, m/z) = 344.1 (M+H⁺).

### #14-15: N,N-dimethyl-N'-(3-(1-(thiophen-4-yl)-1H-pyrazol-4-yl)phenyl)azanesulfonamide

Step 1: Using 2-iodothiophen (1.00 g, 4.21 mmol) as a starting material, it was reacted in a manner similar to Preparation Example 1 to obtain 708 mg of the title compound 3-(1-(thiophen-2-yl)-1H-pyrazol-4-yl)aniline. 1H-NMR (300 MHz, CDCl3): δ 8.39 (s, 1H), 8.09 (s, 1H), 7.89(1H, d, J=8.64 Hz), 7.75 (1H, d, J=8.64 Hz), 7.65 (m, 1H), 7.01 (m, 1H), 6.97 (1H, d, J=7.72 Hz), 6.85 (1H, s), 6.72 (1H, d, J=7.72 Hz), 4.67 (s, 2H); LC-MS (ESI, m/z) = 242.3 (M+H⁺).

Step 2: Using the compound obtained from step 1, 3-(1-(thiophen-2-yl)-1H-pyrazol-4-yl)aniline as a starting material for the reaction, it was reacted in a similar manner to Example #14-3 to obtaine 63 mg of the title compound. 1H-NMR (300 MHz, CDCl3): δ 10.50 (s, 1H), 8.39 (s, 1H), 8.12 (s, 1H), 7.95(1H, d, J=8.64 Hz), 7.79 (1H, d, J=8.64 Hz), 7.64 (m, 1H), 7.01 (m, 1H), 6.98 (1H, d, J=7.72 Hz), 6.85 (1H, s), 6.71 (1H, d, J=7.72 Hz), 6.52 (s, 1H), 2.88 (s, 6H); LC-MS (ESI, m/z) = 348.9 (M+H⁺).

### #14-16: N,N-dimethyl-N'-(3-(1-(4-fluorophenyl)-1H-pyrazol-4-yl)phenyl)azanesulfonamide

Step 1: Using 1-fluoro-4-iodobenzene (1.00 g, 4.51 mmol) as a starting material, it was reacted in a manner similar to Preparation Example 1 to obtain 672 mg of the title compound 3-(1-(4-fluorophenyl)-1H-pyrazol-4-yl)aniline. 1H-NMR (300 MHz, CDCl3): δ 8.27 (s, 1H), 8.08 (s, 1H), 7.82 (2H, d, J=8.74 Hz), 7.68 (2H, d, J=8.74 Hz), 7.46 (m, 1H), 7.04 (1H, d, J=7.82 Hz), 6.85 (1H, s), 6.72 (1H, d, J=7.69 Hz), 4.64 (s, 2H); LC-MS (ESI, m/z) = 254.1 (M+H⁺).

Step 2: Using the compound obtained in step 1, 3-(1-(4-fluorophenyl)-1H-pyrazol-4-yl)aniline (200 mg, 0.78 mmol) as a starting material for the reaction, it was reacted in a similar manner to Example #14-3 to obtaine 65 mg of the title compound. 1H-NMR (300 MHz, CDCl3): δ 10.50 (s, 1H), 8.26 (s, 1H), 8.08 (s, 1H), 7.84 (2H, d, J=8.71 Hz), 7.69 (2H, d, J=8.74 Hz), 7.48 (m, 1H), 7.06 (1H, d, J=7.85 Hz), 6.85 (1H, s), 6.72 (1H, d, J=7.69 Hz), 6.52 (s, 1H), 2.90 (s, 6H); LC-MS (ESI, m/z) = 361.1 (M+H⁺).

### #14-17: N,N-dimethyl-N'-(3-(1-(4-([1,1'-biphenyl]-1H-pyrazol-4-yl)phenyl)azanesulfonamide

Step 1: Using 1-iodobiphenyl (1.00 g, 3.57 mmol) as a starting material, it was reacted in a similar manner to Preparation Example 1 to obtaine 812 mg of the title compound 3-(1-([1,1'-biphenyl]-4-yl)-1H-pyrazol-4-yl)aniline. 1H-NMR (300 MHz, CDCl3): δ 8.32 (s, 1H), 8.14 (s, 1H), 7.81 (2H, d, J=8.77 Hz), 7.72 (2H, d, J=8.77 Hz), 7.69-7.52 (m, 5H), 7.46 (m, 1H), 7.01 (1H, d, J=7.82 Hz), 6.85 (1H, s), 6.78 (1H, d, J=7.66 Hz), 4.65 (s, 2H); LC-MS (ESI, m/z) = 312.1 (M+H⁺).

Step 2: Using the compound obtained from step 1, 3-(1-([1,1'-biphenyl]-4-yl)-1H-pyrazol-4-yl)aniline (200 mg, 0.64 mmol) as a starting material, it was reacted in a similar manner to Example #14-3 to obtaine 61 mg of the title compound. 1H-NMR (300 MHz, CDCl3): δ 10.48 (s, 1H), 8.32 (s, 1H), 8.11 (s, 1H), 7.83 (2H, d, J=8.77 Hz), 7.70 (2H, d, J=8.77 Hz), 7.70-7.52 (m, 5H), 7.46 (m, 1H), 7.03 (1H, d, J=7.74 Hz), 6.81 (1H, s), 6.73 (1H, d, J=7.69 Hz), 6.50 (s, 1H), 2.91 (s. 6H); LC-MS (ESI, m/z) = 419.0 (M+H⁺).

### #14-18: N,N-dimethyl-N'-isobutyl-N'-(3-(1-(4-(trifluoromethyl)phenyl)-1H-pyrazol-4-yl)phenyl)azanesulfonamide

1-iodo-2-methylpropan (53 mg, 0.29 mmol) was added to the starting material #14-3 (100 mg, 0.24 mmol) and it was reacted in a manner similar to Example #14-4 to obtain 32 mg of the title compound. ¹H-NMR (300 MHz, CDCl₃): δ 8.22 (s, 1H), 8.01 (s, 1H), 7.87 (d, 2H, J= 8.62 Hz), 7.71 (d, 2H, J= 8.62 Hz), 7.59 (m, 1H), 7.49 (m, 1H), 7.42 (t, 1H, J= 7.60 Hz), 7.30 (m, 1H), 2.98 (d, J=7.72 Hz, 2H), 2.82 (s, 6H), 1.68 (m, 1H), 0.92 (d, 6H, J=6.8 Hz); LC-MS (ESI, m/z) = 466.9 (M+H⁺).

### #14-19: N,N-dimethyl-N'-allyl-N'-(3-(1-(4-(trifluoromethyl)phenyl)-1H-pyrazol-4-yl)phenyl)azanesulfonamide

3-bromo-1-propene (35 mg, 0.29 mmol) was added to the starting material #14-3 (100 mg, 0.24 mmol) and it was reacted in a similar manner to Example #14-4 to obtain 41 mg of the title compound. ¹H-NMR (300 MHz, CDCl₃): δ 8.23 (s, 1H), 8.04 (s, 1H), 7.86 (d, 2H, J= 8.45 Hz), 7.72 (d, 2H, J= 8.45 Hz), 7.61 (m, 1H), 7.52 (m, 1H), 7.41 (t, 1H, J= 7.63 Hz), 7.31 (m, 1H), 5.85 (m, 1H), 5.19 (d, J=17.1 Hz, 1H), 5.17 (d, J=17.1 Hz, 1H), 3.73 (d, J=6.2 Hz, 1H), 2.82 (s, 6H); LC-MS (ESI, m/z) = 450.9 (M+H⁺).

### #14-20: N,N-dimethyl-N'-phenyl-N'-(3-(1-(4-(trifluoromethyl)phenyl)-1H-pyrazol-4-yl)phenyl)azanesulfonamide

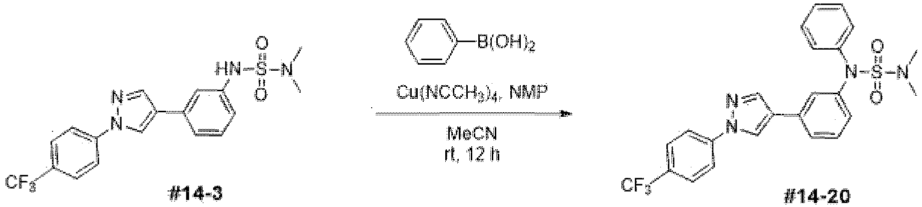

Starting material #14-3 (100 mg, 0.24 mmol), phenylboronic acid (36 mg, 0.29 mmol), Tetrakis(acetonitrile)copper(I) hexafluorophosphate (108 mg, 0.29 mmol), N-methylpiperidine (0.17 mL, 1.46 mmol) were added to anhydrous MeCN and stirred at room temperature for 12 hours. After the reaction product was confirmed by TLC, it was diluted with DCM and washed with NH4Cl solution. The separated organic layer was dehydrated with anhydrous Na2SO4, concentrated under reduced pressure, and separated by column chromatography to obtain 29 mg of the title compound #14-20.

¹H-NMR (300 MHz, CDCl₃): δ 8.23 (S, 1H), 8.08 (s, 1H), 7.88 (d, 2H, J= 8.48 Hz), 7.79 (d, 2H, J= 8.48 Hz), 7.42 (m, 1H), 7.31 (m, 2H), 7.29-7.15 (m, 5H), 7.09 (m, 1H), 2.87 (s, 6H); LC-MS (ESI, m/z) = 486.9 (M+H⁺).

### #14-21: N-(3-(1-(4-(trifluoromethyl)phenyl)-1H-pyrazol-4-yl)phenyl)piperidine-1-sulfonamide

The compound synthesized in Preparation Example 1, 3-(1-(4-(trifluoromethyl)phenyl)-1H-pyrazol-4-yl)aniline (100 mg, 0.33 mmol) and Piperidine-1-sulfonyl chloride (56 uL, 0.39 mmol) was reacted similarly to Example #14-3 to obtain 42 mg of the title compound. ¹H-NMR (300 MHz, CDCl₃): δ 8.23 (S, 1H), 7.98 (s, 1H), 7.84 (d, 2H, J= 8.31 Hz), 7.74 (d, 2H, J= 8.31 Hz), 7.36 (m, 1H), 7.33 (m, 2H), 7.01 (m, 1H), 3.07 (t, J= 7.7 Hz, 4H), 1.53-1.43 (m, 6H); LC-MS (ESI, m/z) = 451.1 (M+H⁺).

### 3. #14-6, #14-7, #14-8 synthesis scheme

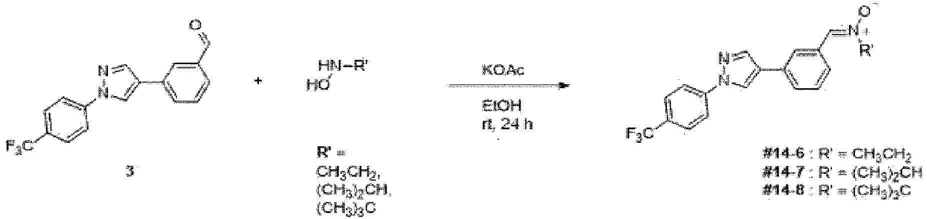

### #14-6: (E)-N-ethyl-1-(3 -(1-(4-(trifluoromethyl)phenyl)-1H-pyrazol-4-yl)phenyl)methanimine oxide

Compound 3 (100 mg, 0.32 mmol) obtained in Preparation Example 2 was placed in a reaction flask and dissolved in 3 mL of anhydrous EtOH. After adding N-methylhydroxylamine.HCl (79 mg, 0.95 mmol) and potassium acetate (93 mg, 0.95 mmol), the mixture was stirred at room temperature for 24 hours. After the reaction product was confirmed by TLC, it was diluted with EtOAc and washed with purified water. The separated organic layer was dehydrated with anhydrous Na₂SO₄, concentrated under reduced pressure, and separated by column chromatography to obtain 76 mg of the title compound #14-6. ¹H-NMR (300 MHz, CDCl₃): δ 8.35 (s, 1H), 8.12 (s, 1H), 7.86 (d, 2H, J= 8.59 Hz), 7.74 (d, 2H, J= 8.59 Hz), 7.70 (m, 1H), 7.56 (m, 1H), 7.44 (t, 1H, J= 7.63 Hz), 7.32 (m, 1H), 3.98 (q, 2H, J=9.00 Hz), 1.56 (t, 3H, J=9.00 Hz); LC-MS (ESI, m/z) = 360.2 (M+H⁺).

### #14-7: (E)-N-isopropyl-1-(3-(1-(4-(trifluoromethyl)phenyl)-1H-pyrazol-4-yl)phenyl)methanimine oxide

Compound 3 (100 mg, 0.32 mmol) obtained in Preparation Example 2 was placed in a reaction flask and dissolved in 3 mL of anhydrous EtOH. After adding N-isopropylhydroxylamine.HCl (105 mg, 0.95 mmol) and potassium acetate (93 mg, 0.95 mmol), the mixture was stirred at room temperature for 24 hours. After the reaction product was confirmed by TLC, it was diluted with EtOAc and washed with purified water. The separated organic layer was dehydrated with anhydrous Na₂SO₄,concentrated under reduced pressure, and separated by column chromatography to obtain 60 mg of the title compound #14-7. ¹H-NMR (300 MHz, CDCl₃): δ 8.32 (s, 1H), 8.12 (s, 1H), 7.86 (d, 2H, J= 8.50 Hz), 7.71 (d, 2H, J= 8.50 Hz), 7.65 (m, 1H), 7.56 (m, 1H), 7.41 (t, 1H, J= 7.61 Hz), 7.29 (m, 1H), 4.21 (m, 1H), 1.51 (s, 3H), 1.49 (s, 3H); LC-MS (ESI, m/z) = 374.0 (M+H⁺).

### #14-8: (E)-N-tert-butyl-1-(3-(1-(4-(trifluoromethyl)phenyl)-1H-pyrazol-4-yl)phenyl)methanimine oxide

Compound 3(100 mg, 0.32 mmol) obtained in Preparation Example 2 was placed in a reaction flask and dissolved in 3 mL of anhydrous EtOH. After adding N-tert-butylhydroxylamine.HCl (118 mg, 0.95 mmol) and potassium acetate (93 mg, 0.95 mmol), the mixture was stirred at room temperature for 24 hours. After the reaction product was confirmed by TLC, it was diluted with EtOAc and washed with purified water. The separated organic layer was dehydrated with anhydrous Na₂SO₄, concentrated under reduced pressure, and separated by column chromatography to obtain 71 mg of the title compound #14-8. ¹H-NMR (300 MHz, CDCl₃): δ 8.38 (s, 1H), 8.21 (s, 1H), 7.84 (d, 2H, J= 8.12 Hz), 7.72 (d, 2H, J= 8.12 Hz), 7.59 (m, 1H), 7.53 (m, 1H), 7.40 (t, 1H, J= 7.61 Hz), 7.27 (m, 1H), 1.60 (s, 9H); LC-MS (ESI, m/z) = 388.1 (M+H⁺).

### 4. #14-9, #14-10 synthetic scheme

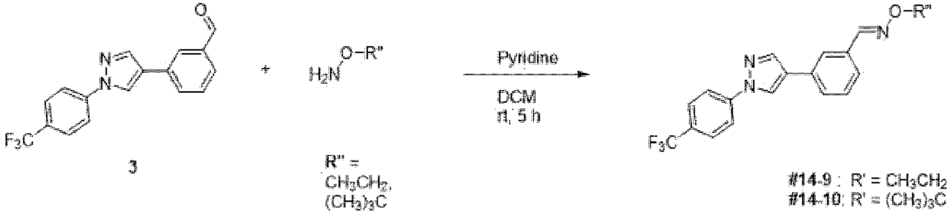

### #14-9: (E)-3-(1-(4-(trifluoromethyl)phenyl)-1H-pyrazol-4-yl)benzaldehyde O-ethyl oxime

Compound 3 (100 mg, 0.32 mmol) obtained in Preparation Example 2 was placed in a reaction flask and dissolved in 5 mL of anhydrous DCM. After adding ethoxyamine.HCl (46 mg, 0.47 mmol) and pyridine (56 µL, 0.70 mmol), the mixture was stirred at room temperature for 5 hours. After the reaction product was confirmed by TLC, it was diluted with EtOAc and washed with purified water. The separated organic layer was dehydrated with anhydrous Na₂SO₄, concentrated under reduced pressure, and separated by column chromatography to obtain 78 mg of the title compound #14-9. ¹H-NMR (300 MHz, CDCl₃): δ 8.46 (s, 1H), 8.32 (s, 1H), 8.08 (s, 1H), 7.80 (d, 2H, J= 8.45 Hz), 7.71 (d, 2H, J= 8.45 Hz), 7.64 (m, 1H), 7.53 (m, 1H), 7.41 (t, 1H, J= 7.61 Hz), 7.32 (m, 1H), 3.57 (q, 2H, J=9.82 Hz), 1.51 (t, 3H, J=9.82 Hz); LC-MS (ESI, m/z) = 360.1 (M+H⁺).

### #14-10: (E)-3-(1-(4-(trifluoromethyl)phenyl)-1H-pyrazol-4-yl)benzaldehyde O-(tert-butyl) oxime

Compound 3 (100 mg, 0.32 mmol) obtained in Preparation Example 2 was placed in a reaction flask and dissolved in 5 mL of anhydrous DCM. After adding tert-butoxyamine.HCl (59 mg, 0.47 mmol) and pyridine (56 µL, 0.70 mmol), the mixture was stirred at room temperature for 5 hours. After confirming the reaction product by TLC, it was diluted with EtOAc and washed with purified water. The separated organic layer was dehydrated over anhydrous Na₂SO₄, concentrated under reduced pressure, and separated by column chromatography to obtain 85 mg of the title compound #14-10. ¹H-NMR (300 MHz, CDCl₃): δ 8.52 (s, 1H), 8.38 (s, 1H), 8.12 (s, 1H), 7.83 (d, 2H, J= 8.42 Hz), 7.74 (d, 2H, J= 8.42 Hz), 7.63 (m, 1H), 7.50 (m, 1H), 7.47 (t, 1H, J= 7.59 Hz), 7.32 (m, 1H), 1.25 (s, 9H); LC-MS (ESI, m/z) = 388.0 (M+H⁺).

### <Example 2> Breast cancer cell line and culture method thereof

Human-derived tumor cell lines and breast cancer cell lines MCF7 and T47D, BT549, MDA-MB231, and MDA-MB453 was cultured in a DMEM medium (Corning, NY, USA), containing 10% fetal bovine serum (FBS; HyClone, South Logan, UT) and 1% penicillin/streptomycin at 37° C and 5% CO₂ conditions.

Thereafter, the cell lines and compounds prepared through the above method were used in the following Examples.

### <Example 3> Confirmation of Cancer Cell-Specific Killing Effect (anticancer effect) of KRCT-1

### 1. Confirmation of cancer cell killing effect (anticancer effect) through cell proliferation and survival analysis of KRCT-1

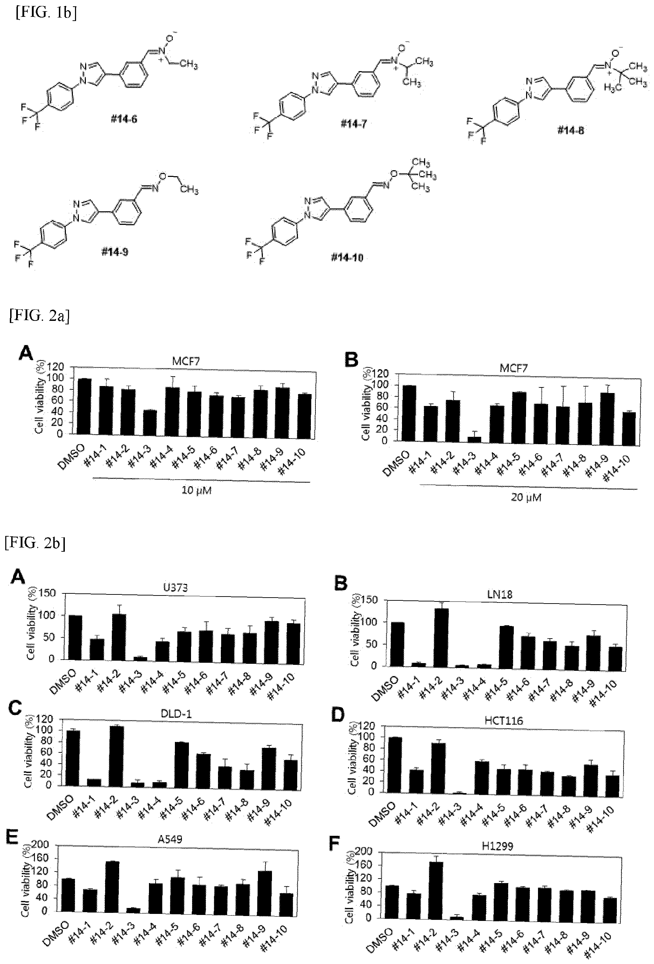

In order to confirm the inhibition of cancer cell proliferation by KRCT-1, WST-8 analysis (Cyto XTM cell viability assay kit, LPS solution, Daejeon, Korea) was performed according to the manufacturer's protocol. Colorectal cancer (DLD1, HCT-116), lung cancer (H1299, A549), brain tumor (U373, LN18), breast cancer (T47D, BT549, MDA-MB 453, MCF7 and MDA MB-231), stomach cancer (SNU668), blood cancer (MV4-11) cells were seeded in 96-well plates, cultured for 24 hours, and then treated with the compound. Then, the absorbance was measured at 450 nm using a Versamax microplate reader (Molecular Devices, CA, USA), and the results of the experiment are shown in FIG. 2 and 3B.

### 2. Confirmation of cancer cell killing effect (anticancer effect) through colonogenic assay of KRCT-1

Colonogenic analysis was performed to determine whether the selected KRCT-1 could efficiently kill cancer cells. First, cells of various breast cancer cell lines BT549, MCF7, T47D and MDA MB231 (500-1000 cells) were cultured in a 60 mm culture plate, and 24 hours later, 10 µM of the compound was treated, respectively, and cultured for 14 days. It was fixed and stained with tryptophan blue added with methanol, quantified using Image J software, and was shown in FIG. 3C.

As can be seen in FIG. 3C, it was confirmed that the number and size of cell colonies were significantly reduced upon KRCT-1 treatment in various breast cancer cell lines. As a result, KRCT-1 effectively kills the colonies of cancer cells.

### 3. Confirmation of cancer cell killing effect (anticancer effect) through tumorsphere forming assay of KRCT-1

In order to determine whether the selected KRCT-1 can effectively reduce the tumorsphere formation in cancer cells, tumorsphere forming assay was performed. Breast cancer cells MCF7 (2500-10000 cells) were suspended in serum-free DMEM/F12 (welGENE), containing 1% penicillin, B-27 (1:50; invitrogen), 20 ng/mL epidermal growth factor (R&D Systems) and 20 ng/mL fibroblasts growth factors (R&D Systems) on low adhesion plates and cultured. After 24 hours had elapsed, the cells were treated with the compound and cultured for 14 days, and then the diameter of the tumorspheres was measured and quantified, which is shown in FIG. 3D.

As confirmed in FIG. 3D, it was confirmed that the diameter of cancer cell tumorspheres was significantly reduced in the case that various breast cancer cell lines were treated with KRCT-1. Consequently, KRCT-1 effectively reduces the formation of tumorspheres in cancer cells.

### <Example 4> Confirmation of Anticancer Effect of KRCT-1 in Immunodeficient Mouse Tumor Model (Xenograft mouse model)

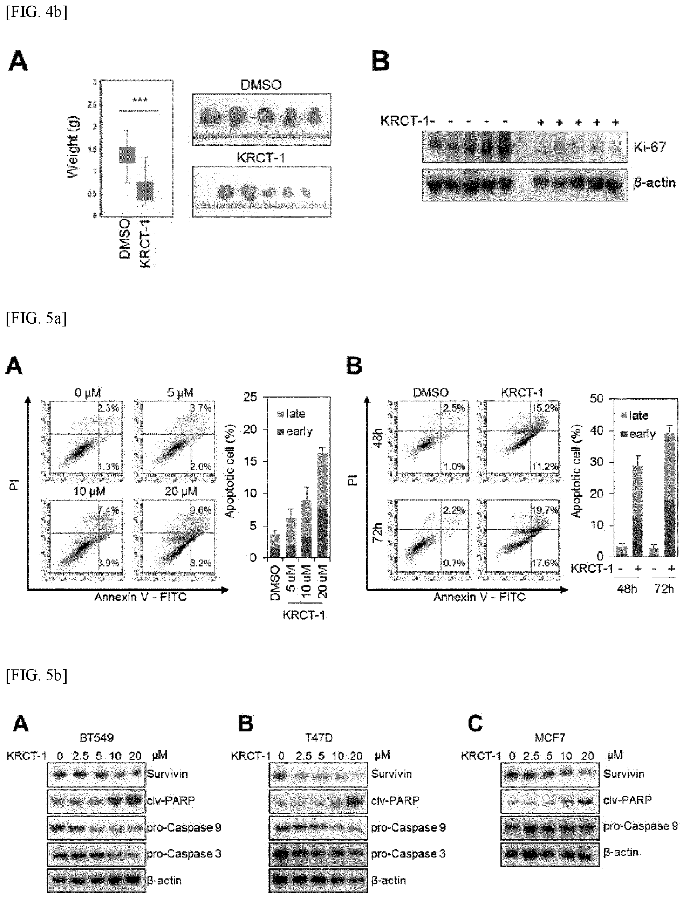

By subcutaneously implanting a cancer cell line into an immunodeficient mouse tumor model, it was attempted to verify whether the anticancer effect of the compound actually occurs at the in vivo level. After the tumor was formed by implanting the BT549 cell line subcutaneously in the thigh of an immunodeficient mouse, in the case that the size of the tumor reached 50~100 mm^~3~, the compound was intraperitoneally injected at 30 mg/kg twice a week. The tumor volume and the mouse weight were measured 3 times a week for the control group and the KRCT-1 treatment group, respectively. A in FIG. 4a and A in FIG. 4b were observed that the group treated with KRCT-1 had a smaller tumor volume and size than the control group when the breast cancer cell line was 4 weeks after transplantation. Western blotting was performed on the isolated mouse tumor for the tumor growth antigen Ki-67, which is shown B in FIG. 4b. B in FIG. 4a shows a result of measuring the weight of the mouse 3 times a week during the administration of the compound.

As shown in FIG. 4, it was confirmed that the KRCT-1 treated group had a small tumor volume and size, and the expression of Ki-67 was significantly reduced. As a result, it was confirmed that the KRCT-1 compound inhibited tumor growth and had an anticancer effect. In addition, since the body weight of immunodeficient mice was not significantly changed with KRCT-1 treatment, it was confirmed that KRCT-1 was not toxic and could inhibit tumor growth.

### <Example 5> Confirmation of anti-cancer effect of KRCT-1

### 1. Confirmation of apoptosis effect through FACS

After Annexin V/PI staining, FACS analysis was performed to confirm the cancer cell killing effect. Cancer cell lines MCF7 and BT549 were treated for 48 h in the presence and absence of 20 µM of KRCT-1, and analysis of Annexin V binding was performed using the Annexin V-FITC apoptosis Detection Kit (BD Biosciences, San Jose, CA, USA) according to the manufacturer's recommended protocol. Briefly, after collecting all cells, including the cell medium, the cells were centrifuged at 1500 rpm for 5 minutes, washed with 1× PBS, resuspended at 1×10⁶ cell concentration, and then 100 µl of the cell suspension was added in a 5 ml tube, and 5 µl of Annexin V-FITC and 5 µl of PI were added thereto and cultured in the dark for 15 minutes at RT. 400 µl of 1× binding buffer was added to each tube and samples were analyzed by flow cytometry. The results of the FACS assay are shown in FIG. 5a. As a result, it was confirmed that MCF7 induced about 40%, BT549 induced about 23% cell death.

### 2. Confirmation of apoptosis effect through confirmation of molecular expression

Apoptosis by KRCT-1 was confirmed through Western blotting. After separation of 20 µg of protein by SDS-PAGE electrophoresis and transferred to PVDF membrane, the following specific antibodies were used: rabbit polyclonal antibodies cleaved PARP (Asp214) (#9541) and phospho-Chk2 (Thr68) ( #2661; Cell Signaling Technology, MA); rabbit monoclonal antibodies caspase3 (#9664), chk2 (#6334S), survivin (#2808S); mouse monoclonal antibody caspase 9 (551246; BD Pharmingen, CA, USA). In addition, mouse monoclonal antibody β-actin (C4; Santa Cruz Biotechnology, CA, USA) was used as a loading control, washed with 1× PBS/0.1% Tween 20, and HRP-binding secondary antibody and improved chemiluminescence detection system was used to detect the binding protein, and an image of the band was obtained using the Amersham Imager 600 system (GE Healthcare Life Science), which is shown in FIG. 5b.

Breast cancer cell lines BT549, T47D, and MCF7 were treated with 0, 2.5, 5, 10, and 20 µM of KRCT-1 in a dose-dependent manner for 24 h each, and the expression level of survivin essential for the survival of cancer cells and the expression levels of an apoptosis markers, clv-PARP, caspase 9 and caspase 3 were checked. According to the concentration of the treated KRCT-1, the expression levels of survivin and pro-caspase 9 in cancer cells were decreased, and it was confirmed that the truncated PARP and caspase3 increased during apoptosis. Through the above results, it was confirmed that apoptosis was activated due to KRCT-1.

### <Example 6> Confirmation of Radiosensitivity Synergistic Effect of Cancer Cell lines Through Treatment of KRCT-1

### 1. Confirmation of combined effects of radiation through clonogenic assay

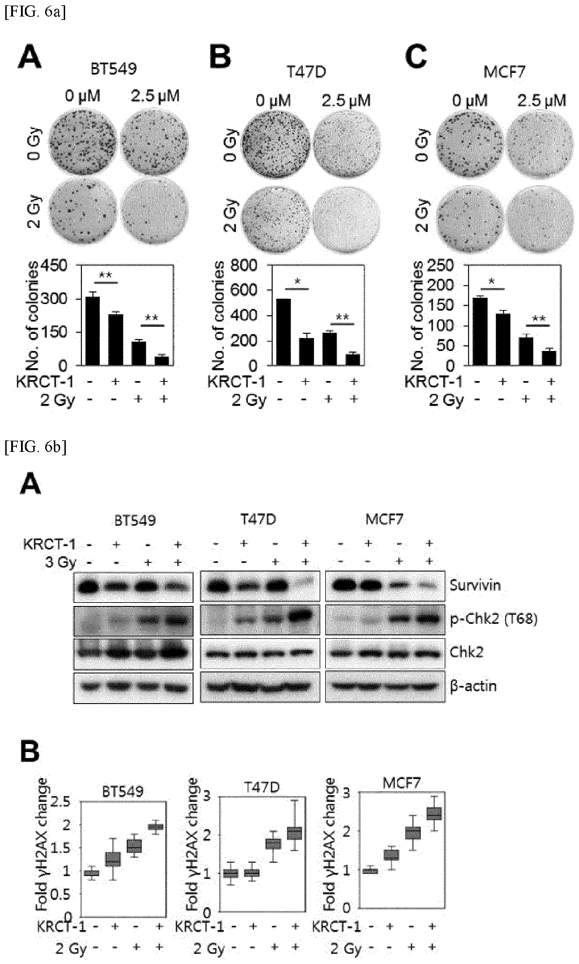

A clonogenic assay was performed to confirm the effect of the combination of KRCT-1 and radiation. To perform clonogenic assay, breast cancer cell lines BT549, T47D and MCF7 were treated with 2.5 µM of KRCT-1 compound, and 2.5 Gy radiation was used using Biobeam GM8000 (Gamma-Gamma-Service Medical GmbH, Leipzig, Germany). The experimental technique for the remaining colony formation was performed in the same manner as in the above Example, and was shown in FIG. 6a. In the group treated with KRCT-1, the radiation treatment effect of the two cancer cell lines was significant compared to the group treated with DMSO, confirming that the number of cancer cell colonies decreased.

### 2. Confirmation of radiation combination effect through Western blotting experiment

In order to confirm the synergistic effect of radiosensitivity of cancer cell lines through treatment with KRCT-1 through Western blotting experiments, 10 µM of KRCT-1 compound was treated in breast cancer cell lines BT549, T47D and MCF7, and Biobeam GM8000 (Gamma-Gamma-Service Medical GmbH, Leipzig, Germany) was used to culture for 30 h after treatment with 3 Gy radiation. For Western blotting, 20 µg of protein was isolated through SDS-PAGE electrophoresis and transferred to a PVDF membrane. The following specific antibodies were used: rabbit polyclonal antibody phospho-Chk2 (Thr68) (#2661; Cell Signaling Technology, MA); rabbit monoclonal antibody chk2 (#6334S) were used. The remaining conditions of Western blotting were performed in the same manner as in the above Example. As a result, as shown in FIG. 6A, the expression level of survivin decreased and the expression level of phosphorylated Chk2 increased in the group treated with KRCT-1 after the radiation treatment. Through the above results, it was confirmed that apoptosis of cancer cells appeared effectively.

### 3. Confirmation of effect of KRCT-1 on DNA damage

To determine how KRCT-1 affects DNA damage induced by radiation, a γ-H2AX foci experiment was performed. In the case that single-stranded or double-stranded DNA is cleaved by various DNA damaging agents including radiation, γ-H2AX phosphorylation occurs and nuclear foci are generated in the cell nucleus. Using this as an index, the effect of the combination according to DNA damage was evaluated. After culturing 2.5×10⁴ MCF7 cells in a 24-well plate for 48 h, KRCT-1 20 µM was treated for 24 h, and the cells were fixed at 1 h after irradiation with 2 Gy of radiation. Then, after washing with phosphate buffered saline for permeation, 4% paraformaldehyde and 0.2% Triton-X were added and reacted for 10 min each. After 1 h pretreatment with 5% fetal bovine serum, 1:100 dilution of γ-H2AX (05-656; EMD Millipore Corp., MA, USA) antibody to phosphorylated H2AX for was treated for 16 h, and it was washed 3 times with 0.1% TritonX-100/phosphate buffered saline solution (Tritonx-100/PBS). Alexa488-conjugated secondary antibody (AP132JA4, Invitrogen) was reacted for 2 h at room temperature and labeled, and phosphorylated γ-H2AX foci were photographed and analyzed with IN Cell Analyzer 6000 (GE Healthcare Life Science). As a result, as shown in FIG. 6B, it was confirmed that DNA damage occurred in relatively many cancer cells in the case that treated with KRCT-1 and radiation compared to each control group, and it was confirmed that the sensitivity to radiation was improved. Taken together, in cancer cells, it was confirmed that KRCT-1 is a material that can be effectively used as a radiosensitizer.

### <Example 7> Confirmation of Trk-A Selective Inhibitory Activity of KRCT-1: Efficacy and Specificity Assay through In Vitro Kinase Assay

To measure the selective reactivity of KRCT-1 to a specific kinase, in vitro kinase assay profiling was commissioned to Goma Biotech (Seoul, Korea), and the enzyme reaction experiment was performed at Eurofins Pharma Discovery Services (Scotland, UK). For kinase profiling, 104 kinase series and 10 µM KRCT-1 were used in the reaction as a broad oncology panel. To obtain the IC₅₀ kinase assay of Trk-A, KRCT-1 serially diluted 3-fold from 100 µM was used.

### <Example 8> Confirmation of Trk-A Inhibitory Activity by KRCT-1 in Cancer Cell Lines

In cancer cell lines, it was confirmed whether KRCT-1 inhibits the activity of Trk-A kinase to inhibit downstream signaling. In the case that Trk-A is activated by ligand, the MEK/ERK and STAT3 signaling pathways in the cell are activated. Using this as an indicator, changes in phosphorylated ERK and phosphorylated STAT3 were confirmed in the case that cancer cell lines were treated with KRCT-1 concentrations (2.5, 5, 10, 20 µM). For Western blotting, 20 µg of protein was isolated through SDS-PAGE electrophoresis and transferred to PVDF membrane. The following specific antibodies were used: rabbit polyclonal antibody phospho-STAT3 (Tyr705) (ab76315; Abcam, UK), mouse monoclonal antibodies phospho-ERK (Thr202/Tyr204) (sc-514302; Santa Cruz Biotechnology), ERK (sc-7383; Santa Cruz Biotechnology), and STAT3 (sc8019; Santa Cruz Biotechnology) were used. The remaining conditions of Western blotting were performed in the same manner as in the above Example.

Although specific parts of the present invention have been described above in detail, it is clear for those skilled in the art that these specific descriptions are merely preferred example embodiments and the scope of the present invention is not limited thereby. Accordingly, the substantial scope of the present invention would be defined by the appended claims and their equivalents.

## Claims

1. A compound represented by Formula 1 below, an isomer thereof, or a pharmaceutically acceptable salt thereof, 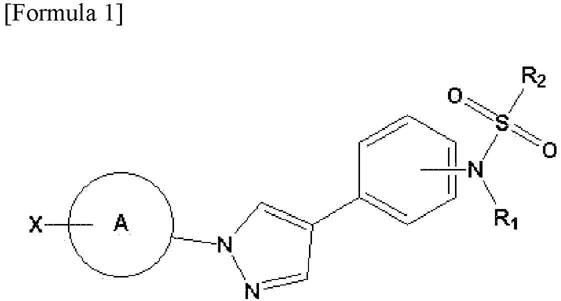
wherein, in Formula 1,
R₁ is selected from hydrogen, (C1~C6)alkyl, (C1~C6)alkene, (C1~C6)alkyne, substituted or unsubstituted aryl(C1~C6)alkyl, or substituted or unsubstituted aryl wherein said aryl may be substituted with 1 to 3 substituents which are independently selected from the group consisting of halo selected from the group consisting of fluoro, chloro and bromo, nitro, cyano, hydroxy, and oxo, R₂ is selected from hydrogen, (C1~C6)alkyl, amino, (C1~C6)alkylamino, or di(C1~C6)alkylamino,
A is any one of aryl, pyridine, thiophene, or biphenyl, and
X is hydrogen, CF₃, CHF₂, CH₂F, (C1~C6)alkyl, (C1~C6)alkoxy, or halo selected from the group consisting of fluoro, chloro, and bromo.

2. A compound represented by Formula 2 below, an isomer thereof, or a pharmaceutically acceptable salt thereof, 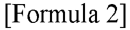 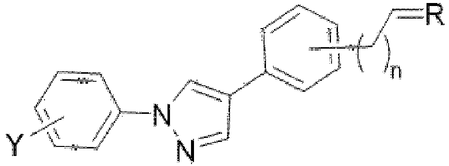 wherein, in Formula 2,
Y is hydrogen, CF₃, CHF₂, CH₂F, (C1~C6)alkyl, (C1~C6)alkoxy, or halo selected from the group consisting of fluoro, chloro, and bromo,
n is an integer between 0 to 5,
R is 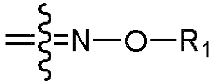 or 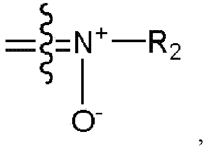 and R₁ and R₂ are each independently selected from hydrogen or substituted or unsubstituted (C1~C6)alkyl, wherein said alkyl may be substituted with 1 to 3 substituents which are independently selected from halo selected from the group consisting of fluoro, chloro, and bromo, or hydroxy.

3. The compound, an isomer thereof, or a pharmaceutically acceptable salt thereof according to claim 1, wherein the compound represented by Formula 1 is any one selected from the group consisting of:
N-methyl-N-(3-(1-(4-(trifluoromethyl)phenyl)-1H-pyrazol-4-yl)phenyl)ethanesulfonamide;
N-(4-chlorobenzyl)-N-(3-(1-(4-(trifluoromethyl) phenyl)-1H-pyrazol-4-yl)phenyl)ethanesulfonamide;
N,N-dimethyl-N'-(3-(1-(4-(trifluoromethyl)phenyl)-1H-pyrazol-4-yl)phenyl)azanesulfonamide;
N,N -dimethyl-N'-methyl-N'-(3-(1-(4-(trifluoromethyl)phenyl)-1H-pyrazol-4-yl)phenyl)azanesulfonamide;
N,N-dimethyl-N'-(4-chlorobenzyl)-N'-(3-(1-(4-(trifluoromethyl)phenyl)-1H-pyrazol-4-yl)phenyl)azanesulfonamide;
N,N-dimethyl-N'-(3-(1-(4-phenyl)-1H-pyrazol-4-yl)phenyl)azanesulfonamide;
N,N-dimethyl-N'-(3-(1-(4-(methyl)phenyl)-1H-pyrazol-4-yl)phenyl)azanesulfonamide;
N,N-dimethyl-N'-(3-(1-( 4-(methoxy)phenyl)-1H-pyrazol-4-yl)phenyl)azanesulfonamide;
N,N-dimethyl-N'-(3-(1-(pyridin-4-yl)-1H-pyrazol-4-yl)phenyl)azanesulfonamide;
N,N-dimethyl-N'-(3-(1-(thiophen-4-yl)-1H-pyrazol-4-yl)phenyl)azanesulfonamide;
N,N-dimethyl-N'-(3-(1-(4-fluorophenyl)-1H-pyrazol-4-yl)phenyl)azanesulfoneamide;
N,N-dimethyl-N'-(3-(1 -(4-([1,1'biphenyl]-1H-pyrazol-4-yl)phenyl)azanesulfonamide;
N,N-dimethyl-N'-isobutyl-N'-(3-(1-(4-(trifluoromethyl)phenyl)-1H-pyrazol-4-yl)phenyl)azanesulfonamide;
N,N-dimethyl-N'-allyl-N'-(3-(1-(4-(trifluoromethyl)phenyl)-1H-pyrazole- 4-yl)phenyl)azanesulfonamide;
N,N-dimethyl-N'-phenyl-N'-(3-(1-(4-(trifluoromethyl)phenyl)-1H-pyrazol-4-yl)phenyl)azanesulfonamide; and
N-(3-(1-(4-(trifluoromethyl)phenyl)-1H-pyrazol-4-yl)phenyl)piperdine-1-sulfonamide.

4. The compound, an isomer thereof, or a pharmaceutically acceptable salt thereof according to claim 2, wherein the compound represented by Formula 2 is any one selected from the group consisting of:
(E)-N-ethyl-1-(3-(1-(4-(trifluoromethyl)phenyl)-1H-pyrazol-4-yl )phenyl)methanimine oxide;
(E)- N-isopropyl-1-(3-(1-(4-(trifluoromethyl)phenyl)-1H-pyrazol-4-yl)phenyl)methanimine oxide;
(E)-N-tert-butyl-1-(3-(1-(4- (trifluoromethyl)phenyl)-1H-pyrazol-4-yl)phenyl)methanimine oxide;
(E)-3-(1-(4-(trifluoromethyl)phenyl)-1H-pyrazol-4-yl)benzaldehyde O-ethyl oxime; and
(E)-3-(1-(4-(trifluoromethyl)phenyl)-1H-pyrazol-4-yl)benzaldehyde O-(tert-butyl) oxime.

5. A pharmaceutical composition for preventing or treating cancer, comprising the compound, an isomer thereof, or a pharmaceutically acceptable salt thereof according to any one of claims 1 to 4, as an active ingredient.

6. The pharmaceutical composition according to claim 5, wherein the cancer is selected from the group consisting of brain tumor, benign astrocytoma, malignant astrocytoma, pituitary adenoma, meningioma, cerebral lymphoma, oligodendroglioma, intracranioma, ependymoma, brainstem tumor, head and neck tumor, laryngeal cancer, oropharyngeal cancer, nasal/sinus cancer, nasopharyngeal cancer, salivary gland cancer, hypopharyngeal cancer, thyroid cancer, chest tumor, small cell lung cancer, non-small cell lung cancer, thymus cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colorectal cancer, anal cancer, bladder cancer, kidney cancer, male genital tumor, penile cancer, urethral cancer, prostate cancer, female genital tumor, cervical cancer, endometrial cancer, ovarian cancer, uterine sarcoma, vaginal cancer, female external reproductive cancer, female urethral cancer, skin cancer, myeloma, leukemia, and malignant lymphoma.

7. The pharmaceutical composition according to claim 5, wherein the pharmaceutical composition is administered in combination with radiation irradiation during cancer treatment.

8. The pharmaceutical composition according to claim 5, wherein the pharmaceutical composition increases sensitivity to radiation of cancer cells or cancer cells having radioresistance.

9. A health functional food composition for preventing or alleviating cancer, containing the compound, an isomer thereof, or a pharmaceutically acceptable salt thereof according to any one of claims 1 to 4, as an active ingredient.

10. A radiosensitizer pharmaceutical composition for treating cancer with radiotherapy, containing the compound, an isomer thereof, or a pharmaceutically acceptable salt thereof according to any one of claims 1 to 4, as an active ingredient.

11. The radiosensitizer pharmaceutical composition for treating cancer with radiotherapy according to claim 10, wherein the cancer is selected from the group consisting of brain tumor, benign astrocytoma, malignant astrocytoma, pituitary adenoma, meningioma, cerebral lymphoma, oligodendroglioma, intracranioma, ependymoma, brainstem tumor, head and neck tumor, laryngeal cancer, oropharyngeal cancer, nasal/sinus cancer, nasopharyngeal cancer, salivary gland cancer, hypopharyngeal cancer, thyroid cancer, chest tumor, small cell lung cancer, non-small cell lung cancer, thymus cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colorectal cancer, anal cancer, bladder cancer, kidney cancer, male genital tumor, penile cancer, urethral cancer, prostate cancer, female genital tumor, cervical cancer, endometrial cancer, ovarian cancer, uterine sarcoma, vaginal cancer, female external reproductive cancer, female urethral cancer, skin cancer, myeloma, leukemia, and malignant lymphoma.

12. The radiosensitizer pharmaceutical composition for treating cancer with radiotherapy according to claim 10, wherein the pharmaceutical composition is administered in combination with radiation irradiation during cancer treatment.
